# EUROPEAN PATENT SPECIFICATION

(11) **EP 3 886 881 B1**
(45) Date of publication and mention of the grant of the patent: **16.07.2025**
(21) Application number: 19816580.5
(22) Date of filing: 02.12.2019
(51) Int. Cl.: A61K 35/74, A61P 5/48, A61P 5/50, A61P 35/00, A61P 35/02

(54) **PREVOTELLA MELANINOGENICA STRAIN FOR USE AGAINST CANCER OR DIABETES**
PREVOTELLA MELANINOGENICA STAMM ZUR VERWENDUNG GEGEN KREBS ODER DIABETES
SOUCHE DE PREVOTELLA MELANINOGENICA POUR SON UTILISATION CONTRE LE CANCER OU LE DIABÈTE

(30) Priority: 30.11.2018 EP 18209623
(43) Date of publication of application: 06.10.2021
(73) Proprietor: Ospedale San Raffaele S.r.l., 20132 Milano (IT)
(72) Inventor: BELLONE, Matteo Maria Salvatore, 20132 Milano (MI) (IT); BREVI, Arianna, 20132 Milano (MI) (IT); CALCINOTTO, Arianna, 20132 Milano (MI) (IT); FERRARESE, Roberto, 20132 Milano (MI) (IT); CANDUCCI, Filippo, 20132 Milano (MI) (IT)
(74) Representative: Pace Napoleone, Maria
(86) International application number: PCT/EP2019/083261
(87) International publication number: WO 2020/109620

(56) References cited:
- WO-A1-2014/182632
- WO-A1-2018/152306
- WO-A1-2019/051381
- WO-A1-2020/097483
- WO-A1-2020/257248
- WO-A2-2020/058979
- BELLONE MATTEO ET AL: "Microbiota-Propelled T Helper 17 Cells in Inflammatory Diseases and Cancer", MICROBIOLOGY AND MOLECULAR BIOLOGY REVIEWS, 20 May 2020 (2020-05-20), United States, XP093139586, [retrieved on 20240311], DOI: 10.1128/MMBR.00064-19
- PRABHALA RAO H. ET AL: "Elevated IL-17 produced by Th17 cells promotes myeloma cell growth and inhibits immune function in multiple myeloma", BLOOD, vol. 115, no. 26, 1 July 2010 (2010-07-01), US, pages 5385 - 5392, XP093139590, ISSN: 0006-4971, Retrieved from the Internet <URL:https://watermark.silverchair.com/zh802610005385.pdf?token=AQECAHi208BE49Ooan9kkhW_Ercy7Dm3ZL_9Cf3qfKAc485ysgAABGQwggRgBgkqhkiG9w0BBwagggRRMIIETQIBADCCBEYGCSqGSIb3DQEHATAeBglghkgBZQMEAS4wEQQM5z7justTAD476m16AgEQgIIEF-1927mH3uqmvo1wmWxL95w_7AmT8Y_nw2aGu-e_FbokACdBu_fO6YkYwORU43nXYJqrpYzuugBagBTfLfyVY> DOI: 10.1182/blood-2009-10-246660
- CALCINOTTO ARIANNA ET AL: "Microbiota-driven interleukin-17-producing cells and eosinophils synergize to accelerate multiple myeloma progression", NATURE COMMUNICATIONS, vol. 9, no. 1, 3 December 2018 (2018-12-03), UK, XP093139588, ISSN: 2041-1723, Retrieved from the Internet <URL:https://www.nature.com/articles/s41467-018-07305-8> DOI: 10.1038/s41467-018-07305-8
- WANG LIN ET AL: "IL-17 can promote tumor growth through an IL-6-Stat3 signaling pathway", JOURNAL OF EXPERIMENTAL MEDICINE, vol. 206, no. 7, 6 July 2009 (2009-07-06), US, pages 1457 - 1464, XP093139591, ISSN: 0022-1007, Retrieved from the Internet <URL:https://rupress.org/jem/article-pdf/206/7/1457/1902105/jem_20090207.pdf> DOI: 10.1084/jem.20090207
- ARIANNA CALCINOTTO ET AL: "Microbiota-driven interleukin-17-producing cells and eosinophils synergize to accelerate multiple myeloma progression", NATURE COMMUNICATIONS, vol. 9, no. 1, 1 December 2018 (2018-12-01), XP055665200, DOI: 10.1038/s41467-018-07305-8
- ERIC V. MARIETTA ET AL: "Suppression of Inflammatory Arthritis by Human Gut-Derived Prevotella histicola in Humanized Mice : SUPPRESSION OF INFLAMMATORY ARTHRITIS BY PREVOTELLA HISTICOLA", ARTHRITIS & RHEUMATOLOGY (HOBOKEN), vol. 68, no. 12, 28 November 2016 (2016-11-28), US, pages 2878 - 2888, XP055665204, ISSN: 2326-5191, DOI: 10.1002/art.39785

## Description

### FIELD OF THE INVENTION

The present invention relates to the field of bacterial strain to be used in medicine. In particular, it relates to the prevention and/or treatment of cancer or diabetes, bacterial strain is *Prevotella melaninogenica.*

### BACKGROUND OF THE INVENTION

While many factors regulating cancer progression are tumor cell autonomous, they are insufficient to induce progression to malignancy. Among the cell-extrinsic drivers of cancer, a strong link has been proposed between diet, commensal bacteria and aerodigestive tract malignancies¹. Microbes within the gut also contribute to carcinogenesis at mucosal sites by altering the balance of epithelial cell proliferation and death, by favoring the production of toxic metabolites from host-produced factors and drugs, and by promoting chronic inflammation and/or local immune suppression .

As the microbiome of each organ is distinct, the effects on inflammation and carcinogenesis are likely to be organ specific². Nevertheless, gut commensal bacteria are involved in the pathogenesis of extramucosal autoimmune diseases³, thus supporting the role of the gut microbiota in shaping systemic immune responses. Yet, the mechanisms by which non-pathogenic microbes drive non-aerodigestive tract malignancies remain to be elucidated.

Commensal bacteria are involved in the differentiation of Th17 cells⁴, which mainly produce IL-17A (also defined IL-17), IL-17F, and IL-22, all cytokines playing a critical role in inflammation⁵. The role of Th17 cells in cancer is controversial. While some authors showed that Th17 cells were efficient in eliminating tumors⁶, others reported accumulation of Th17 cells in several tumors, in which they promoted tumor initiation⁷ and progression⁸.

In multiple myeloma (MM), a B cell neoplasm characterized by the accumulation of clonal plasma cells within the bone marrow (BM), and in most cases a monoclonal protein (i.e., M-spike) in blood and/or urine, Th17 cells have been linked to advanced disease with bone lesions⁹. Of relevance, IL-17 can promote tumor growth through an IL-6-STAT3 signaling pathway¹⁰, which is also pivotal for plasma cell growth¹¹, thus suggesting a role for IL-17 in different phases of MM.

No data are available on the potential role of IL-17-producing cells in the early, asymptomatic phases of MM, and on the mechanisms by which IL-17-producing cells are induced and/or recruited in the BM of MM patients. Smoldering multiple myeloma (SMM) is an asymptomatic phase that may anticipate full-blown MM. The definition of SMM has been proposed to fill the gray zone between monoclonal gammopathy of undetermined significance (MGUS), a rather common finding in the elders, and active MM. Indeed, patients affected by SMM are subjected to more frequent follow-up than MGUS because they have a much higher risk of progression¹². However, likely because of heterogeneity in the pathobiology of the disease and lack of adequate risk stratification, few interventional studies in SMM patients have shown improved overall survival with therapy¹³. Indeed, most of the accepted clinical parameters to define high-risk SMM are evidence-based¹³. This paradigm would benefit from a shift that focuses more on the early modifications in the cellular and molecular composition of the BM microenvironment, thus to identify biological culprits of aggressiveness.

Inventors selected MM as a prototypic extramucosal cancer, and investigated here the potential link between gut microbiota, IL-17 and the progression from asymptomatic SMM to active MM.

The gut microbiota has been causally linked to cancer, yet how intestinal microbes influence progression of extramucosal tumors is poorly understood.

Some bacterial strains have been shown to present therapeutic activity. For instance WO2014196913 refers to a product for use in the treatment of obesity, metabolic syndrome, type 2 diabetes, cardiovascular diseases, dementia, Alzheimer's disease and inflammatory bowel disease comprising at least one isolated bacterial strain from the species Prevotellaceae, wherein the strain is selected from the group consisting of Prevotella copri, Prevotella stercorea, Prevotella histicola, Prevotella ruminicola, Prevotella Bryantii 25A and Prevotella distasonis.

WO2018075886 refers to compositions (e.g., probiotic, therapeutics, pharmaceutical, etc.) comprising one or more strains of bacteria from the families Prevotellaceae, Rikenellaceae, Porphyromonadaceae, Lactobacillaceae, Ruminococcaceae, Lachnospiraceae, and/or Bacteroidaceae and methods of use thereof for inducing immune system maintenance and/or rescuing animals from sepsis.

However, there is still the need for a therapeutic treatment for cancer or diabetes.

### SUMMARY OF THE INVENTION

The present invention is based on the surprising finding that *Prevotella melaninogenica* delays the onset of cancer, in particular of multiple myeloma (MM) in mice injected with Vk12598 cell line. Then, such bacteria delays and/or prevents the development of MM in patients affected by by monoclonal gammopathy of undetermined significance (MGUS) or smoldering-MM (SMM). The bacteria is also effective at treating conditions in which Th17 cells are pathogenic.

The present invention is therefore advantageous to delay and/or inhibit the progression to MM in patients affected by SMM, in particular by oral administration of *Prevotella melaninogenica* or derivatives of this bacterium such as bacterial lysate, bacterial metabolites or antigens and bacterial liquid culture supernatant.

Further, the inventors provide evidence that *Prevotella heparinolytica* promotes the differentiation of Th17 cells colonizing the gut and migrating to the bone marrow (BM) of transgenic Vk*MYC mice, where they favor progression of multiple myeloma (MM). Lack of IL-17 in Vk*MYC mice, or disturbance of their microbiome delayed MM appearance. Similarly, in smoldering MM patients, higher levels of BM IL-17 predicted faster disease progression. IL-17 induced STAT3 phosphorylation in murine plasma cells and activated eosinophils. Treatment of Vk*MYC mice with antibodies blocking IL-17, IL-17RA and IL-5 reduced BM accumulation of Th17 cells and eosinophils and delayed disease progression. Thus, in Vk*MYC mice, commensal bacteria unleash a paracrine signaling network between adaptive and innate immunity that accelerates progression to MM and can be targeted by already available therapies.

Further, it was surprisingly found that administration of *Prevotella melaninogenica* delayed the appearance of diabetes when compared to mice receiving oral gavage of PBS.

The present findings support the modulation of the gut microbiota to restrain Th17 skew in all those pathologies in which Th17 and IL17 are pathogenic. The invention is set out in the appended set of claims. Then, the present invention provides at least one bacterial strain of *Prevotella melaninogenica* or a part thereof for for use in the treatment and/or prevention of cancer or for use in the prevention of diabetes, preferably type I diabetes and
wherein said cancer is caused by a tumor cell expressing IL-17 or the receptor for IL-17.

Preferably the cancer is selected from the group consisting of: multiple myeloma (MM), bladder cancer, brain and CNS cancer, breast cancer, cervical cancer, colorectal cancer, esophageal cancer, gastric cancer, gastro-intestinal cancer, head and neck cancer, kindey cancer, liver cancer, lung cancer, lymphoma, ovarian cancer, pancreatic cancer, prostate cancer, chronic lymphocytic leukemia.

Preferably the at least one bacterial strain of *Prevotella melaninogenica* or a part thereof delays the development of MM in a subject affected by monoclonal gammopathy of undetermined significance (MGUS) or smoldering MM (SMM).

Preferably the strain is isolated from a biological sample, alive, sporulated, encapsulated, genetically modified or lyophilized.

The present invention also provides a composition comprising at least one bacterial strain of *Prevotella melaninogenica* or a part thereof as defined above and at least one pharmaceutical acceptable carrier, wherein said composition is for use in the treatment of cancer or in the prevention of diabetes. Preferably the cancer indicated above.

Preferably the composition comprises at least 1 × 10⁴ *Prevotella melaninogenica,* preferably between 1×10⁷ to 1×10¹² *Prevotella melaninogenica.*

Preferably the strain of *Prevotella melaninogenica* is *Prevotella melaninogenica* isolated from a biological sample of a subject (stool, blood, urine ect) or is deposited with the accession number DSM 7089 or DSM-26980 in the DSMZ bank.

In a preferred embodiment the composition is a nutraceutical or a food.

The invention also describes a method for the prognosis of cancer, comprising the steps of:
a) measuring the amount of IL-17 in a biological sample isolated from a subject
b) comparing said measured IL-17 level to a control IL-17 amount.

Preferably said cancer is multiple myeloma or gastrointestinal cancer. Preferably the biological sample is a bone marrow sample or a gastric biopsy.

Preferably the control amount is the amount of IL-17 in a biological of an healthy subject. The amount of IL-17 may be measured by any known methods in the art such as ELISA assays, bead-based arrays such as Bio-Plex^{®} Multiplex Immunoassay System from Biorad or Multiplex Luminex^{™} Protein Assays from ThermoFisher scientific, or Cytometric Bead Array from BD Biosciences, antibody arrays such as Proteome Profiler Human Cytokine Array from R&D Systems. Preferably in the present method, if the measured amount of IL-17 is higher than the control amount, the subject is at high risk of developing MM and/or the progression to MM is fast.

In the present invention a part of *Prevotella melaninogenica* means a lysate of *Prevotella melaninogenica,* metabolites of *Prevotella melaninogenica,* antigens of *Prevotella melaninogenica* or bacterial body component of *Prevotella melaninogenica* such as wall, plasma membrane or *Prevotella melaninogenica* culture supernatant, in particular liquid culture supernatant.

The present invention provides compositions (probiotic, therapeutics, pharmaceutical, ect) comprising one or more strains of *Prevotella melaninogenica.*

In some embodiments, a bacterial composition as described herein may include bacteria as described herein, present in treated fecal material from a healthy donor or individual. Such bacterial compositions may be "directly isolated" and not resulting from any culturing or other process that results in or is intended to result in replication of the population after obtaining the fecal material. In some embodiments, bacteria as described herein include bacterial spores.

In some embodiments, a bacterial composition as described herein may include human bacterial strains. In alternative embodiments, a bacterial composition as described herein may include bacterial strains not generally found in humans.

In some embodiments, a bacterial composition as described herein may include bacteria capable of colonizing the gut of a subject receiving the bacterial composition.

In some embodiments, a bacterial composition as described herein may include live bacteria. In some embodiments, a bacterial composition as described herein may include substantially pure bacteria of the genus *Prevotella,* in particular *Prevotella melaninogenica.* By "substantially pure" or "isolated" is meant bacteria of the genus *Prevotella* that is separated from the components that naturally accompany it, in for example, fecal matter or in the gut. Typically, a bacterial composition as described herein is substantially pure when it is at least 50%, 60%, 70%, 75%, 80%, or 85%, or over 90%, 95%, or 99% by weight, of the total material in a sample. A substantially pure bacterial composition, as described herein, can be obtained, for example, by extraction from a natural source, such as fecal material from a healthy individual, or from bacterial cultures, for example, cultures of any of the bacteria described herein, such as *Prevotella melaninogenica.*

Bacterial compositions, as described herein, may be used to alter the gut microbiota, to populate the gastrointestinal tract, or to diagnose or treat cancer or of a condition associated with IL-17 and/or eosinophils in a subject in need thereof.

By "populating the gastrointestinal tract" is meant establishing a healthy state of the microbiota or microbiome in a subject. In some embodiments, populating the gastrointestinal tract includes increasing or decreasing the levels of specific bacteria in the gastrointestinal tract of a subject. In some embodiments, populating the gastrointestinal tract includes increasing the levels of the bacteria described herein in the gastrointestinal tract of a subject.

By "altering the gut microbiota" is meant any change, either increase or decrease, of the microbiota or microbiome in a subject. In some embodiments, altering the gut microbiota includes increasing or decreasing the levels of specific bacteria, such as *Prevotella melaninogenica* such as in the gastrointestinal tract of a subject. In some embodiments, altering the gut microbiota includes increasing the levels of the bacteria described herein in the gastrointestinal tract of a subject.

By "increase," "increasing", "decrease" or "decreasing" is meant a change in the levels of specific bacteria in the gastrointestinal tract of a subject. An increase or decrease may include a change of any value between 10% and 100%, or of any value between 30% and 60%, or over 100%, for example, a change of about 10%, 20% 30%, 40%, 50%, 60%, 70%, 80%, 90%, 95% or more, when compared to a control. In some embodiments, the increase or decrease may be a change of about 1-fold, 2-fold, 5-fold, 10-fold, 100-fold, or more, when compared to a control.

"Microbiota" refers to the community of microorganisms that occur (sustainably or transiently) in and on an animal subject, typically a mammal such as a human, including eukaryotes, archaea, bacteria, and viruses (including bacterial viruses, such as phage).

"Microbiome" refers to the genetic content of the communities of microbes that live in and on the human body, both sustainably and transiently, including eukaryotes, archaea, bacteria, and viruses (including bacterial viruses, such as phage), where "genetic content" includes genomic DNA, RNA such as ribosomal RNA, the epigenome, plasmids, and other types of genetic information.

The terms "treatment," "treating" or "therapy" encompass prophylactic, palliative, therapeutic, and nutritional modalities of administration of the bacterial compositions described herein. Accordingly, treatment includes amelioration, alleviation, reversal, or complete elimination of one or more of the symptoms in a subject diagnosed with, or known to have, cancer or of a condition associated with IL-17 and/or eosinophils or be considered to derive benefit from the alteration of gut microbiota. In some embodiments, treatment includes reduction of one or more symptoms of gut dysbiosis, asthma, allergy, or atopy by 10%, 20% 30%, 40%, 50%, 60%, 70%, 80%, 90%, 95% or more. Treatment also includes prevention or delay of the onset of one or more symptoms of cancer or of a condition associated with IL-17 and/or eosinophils.

As used herein, a subject may be a mammal, such as a human, non-human primate (e.g., monkey, baboon, or chimpanzee), rat, mouse, rabbit, guinea pig, gerbil, hamster, cow, horse, pig, sheep, goat, dog, cat, etc. In some embodiments, the subject is a patient. The subject may be an infant, such as a human infant less than one year old, or less than three months old. In some embodiments, the subject may be a human infant at any age from 1 day to 350 days old, such as 1 day, 10 days, 20 days, 30 days, 40 days, 50 days, 60 days, 70 days, 80 days, 90 days, 100 days, 1 10 days, 120 days, 130 days, 140 days, 150 days, 160 days, 170 days, 180 days, 190 days, 200 days, 210 days, 220 days, 230 days, 240 days, 250 days, 260 days, 270 days, 280 days, 290 days, 300 days, 310 days, 320 days, 330 days, 340 days, or 350 days old. In some embodiments, the subject may be a fetus. In some embodiments, the subject may be a female, such as a pregnant female. In some embodiments, the subject may be a pregnant female with a family history of asthma, atopy, allergy or gut dysbiosis. In some embodiments, the subject may have undergone, be undergoing, or about to undergo, antibiotic therapy. The subject may be a clinical patient, a clinical trial volunteer, an experimental animal, etc. The subject may be suspected of having or at risk for cancer or of a condition associated with IL-17 and/or eosinophils; be diagnosed with cancer or of a condition associated with IL-17 and/or eosinophils; or be a control subject that is confirmed to not have cancer or of a condition associated with IL-17 and/or eosinophils. Diagnostic methods for cancer or of a condition associated with IL-17 and/or eosinophils, and the clinical delineation of such diagnoses are known to those of ordinary skill in the art. In some embodiments, the subject may be an individual considered to be benefitted by the alteration of gut microbiota. In some embodiments, the subject may be an individual considered to be benefitted by population of the gastrointestinal tract.

### Pharmaceutical & Nutritional Compositions, Dosages & Administration

Bacterial compositions, as described herein, can be provided alone or in combination with other compounds or compositions, in the presence of a carrier, in a form suitable for administration to a subject, as described herein. Where the subject is a fetus, a bacterial composition as described herein may be administered to the mother (i.e., the subject may be a pregnant female).

In some embodiments, a bacterial composition, as described herein, may be a therapeutic, prophylactic, nutritional or probiotic composition.

In some embodiments, a bacterial composition may be a therapeutic, prophylactic, nutritional or probiotic composition including the bacteria of the genus *Prevotella,* in particular *Prevotella melaninogenica.*

In some embodiments, a bacterial composition may be a therapeutic, prophylactic, nutritional or probiotic composition including *Prevotella melaninogenica.*

If desired, a bacterial composition as described herein may be combined with more traditional and existing therapies for cancer or of a condition associated with IL-17 and/or eosinophils.

In some embodiments, a bacterial composition as described herein may be combined with one or more therapies for cancer or of a condition associated with IL-17 and/or eosinophils.

In some embodiments, a bacterial composition as described herein may be administered to a subject prior to, during, or subsequent to treatment with an antibiotic. In some embodiments, a bacterial composition as described herein may be combined with one or more antibiotic including, without limitation, streptomycin, ampicillin, amoxicillin, imipenem, piperacillin/tazobactam, ciprofloxacin, tetracyclines, chloramphenicol or ticarcillin.

The term probiotic herein is intended to mean one or more, or a mixture of, microorganisms that provide health benefits when consumed.

The bacterial compositions can be provided chronically or intermittently.

"Chronic" administration refers to administration of the agent(s) in a continuous mode as opposed to an acute mode, so- as to maintain the initial therapeutic effect (activity) for an extended period of time. "Intermittent" administration is treatment that is not consecutively done without interruption, but rather is cyclic in nature.

Conventional pharmaceutical or nutraceutical practice may be employed to provide suitable formulations or compositions to administer a bacterial composition, as described herein, to subjects suffering from or presymptomatic for cancer or a condition associated with IL-17 and/or eosinophils. Any appropriate route of administration may be employed, for example, dermal, intranasal, inhalation aerosol, topical, gavage, rectal or oral administration.

The bacterial compositions can be in a variety of forms. These forms include, e.g., liquid, semi-solid and solid dosage forms, such as liquid solutions (e.g., injectable and infusible solutions), dispersions or suspensions, tablets, pills, powders, liposomes and suppositories. The preferred form depends, in part, on the intended mode of administration and application. Formulations may be in the form of liquid solutions or suspensions; for oral administration, formulations may be in the form of tablets or capsules; for pediatric oral administration, formulations may be in the form of liquids or suspensions; or for intranasal formulations, in the form of powders, nasal drops, or aerosols. The formulation may be a slow release formulation. In some embodiments, bacterial as described herein, can be formulated as pediatric formulations, such as liquid suspensions.

Bacterial compositions, as described herein, can be formulated as a nutraceutical composition, such as medical foods, nutritional or dietary supplements, food products or beverage products, and include a nutraceutically acceptable carrier. As used herein, a "nutraceutically acceptable carrier" refers to, and includes, any and all solvents, dispersion media, coatings, antibacterial and antifungal agents, isotonic and absorption delaying agents, and the like that are physiologically compatible. The compositions can include a nutraceutically acceptable salt, e.g., an acid addition salt or a base addition salt. In some embodiments, the nutraceutically acceptable carrier is suitable for pediatric use.

Bacterial compositions, as described herein, can be formulated as a pharmaceutical composition and include a pharmaceutically acceptable carrier. As used herein, a "pharmaceutically acceptable carrier" refers to, and includes, any and all solvents, dispersion media, coatings, antibacterial and antifungal agents, isotonic and absorption delaying agents, and the like that are physiologically compatible. The compositions can include a pharmaceutically acceptable salt, e.g., an acid addition salt or a base addition salt. In some embodiments, the pharmaceutically acceptable carrier is suitable for pediatric use. Methods for making formulations are well known in the art. Formulations for parenteral administration may, for example, contain excipients, sterile water, or saline, polyalkylene glycols such as polyethylene glycol, oils of vegetable origin, or hydrogenated napthalenes. Biocompatible, biodegradable lactide polymer, lactide/glycolide copolymer, or polyoxyethylene-polyoxypropylene copolymers may be used to control the release of the compounds. Other potentially useful parenteral delivery systems for include ethylene-vinyl acetate copolymer particles, osmotic pumps, implantable infusion systems, and liposomes. Formulations for inhalation may contain excipients, for example, lactose, or may be aqueous solutions containing, for example, polyoxyethylene-9-lauryl ether, glycocholate and deoxycholate, or may be oily solutions for administration in the form of nasal drops, or as a gel. For therapeutic or prophylactic compositions, the compounds are administered to a subject in an amount sufficient to stop or slow cancer or a condition associated with IL-17 and/or eosinophils.

An "effective amount" of a bacterial composition according to the invention includes an amount sufficient to colonize the gut of a subject for a suitable period of time as determined, for example, by detecting the presence of one or more bacteria of the starin *Prevotella melaninogenica* in a sample, such as a fecal sample, from the subject at specific periods after administration.

In some embodiments, an effective amount includes a therapeutically effective amount or a prophylactically effective amount. A "therapeutically effective amount" refers to an amount effective, at dosages and for periods of time necessary, to achieve the desired therapeutic result, such as treatment, prevention, or amelioration of cancer or of a condition associated with IL-17 and/or eosinophils. A therapeutically effective amount of a bacterial composition may vary according to factors such as the disease state, age, sex, and weight of the subject, and the ability of the bacterial composition to elicit a desired response in the individual. Dosage regimens may be adjusted to provide the optimum therapeutic response. A therapeutically effective amount is also one in which any toxic or detrimental effects of the bacterial composition are outweighed by the therapeutically beneficial effects. A "prophylactically effective amount" refers to an amount effective, at dosages and for periods of time necessary, to achieve the desired prophylactic result, such as treatment, prevention, or amelioration of cancer or of a condition associated with IL-17 and/or eosinophils. Typically, a prophylactic dose is used in subjects prior to or at an earlier stage of disease, so that a prophylactically effective amount may be less than a therapeutically effective amount.

A "probiotic" amount refers to an amount effective, at dosages and for periods of time necessary, to achieve the desired result, such as population of the gastrointestinal tract of a subject after, for example, antibiotic treatment, to normal levels. Typically, probiotic doses are administered at larges excess and may be significantly higher than prophylactically effective or therapeutically effective amounts.

A suitable range for therapeutically or prophylactically effective amounts, or probiotic amounts, of a bacterial composition, as described herein, may include without limitation at least about 10⁶, 10⁷ 10⁸, 10⁹, 10¹⁰, 10¹¹, 10¹², 10¹³ or 10¹⁴ colony forming units (cfus) of the bacteria, per unit dosage.

In some embodiments, dosages for live bacteria, in vegetative or spore forms, can be about 1 ug to about 1000 mg, such as about 0.5 mg to about 5 mg, about 1 mg to about 1000 mg, about 2 mg to about 200 mg, about 2 mg to about 100 mg, about 2 mg to about 50 mg, about 4 mg to about 25 mg, about 5 mg to about 20 mg, about 10 mg to about 15 mg, about 50 mg to about 200 mg, about 200 mg to about 1000 mg, or about 1, 2, 3, 4, 5 or more than g per dose or composition; or 0.001 mg to 1 mg, 0.5 mg to 5 mg, 1 mg to 1000 mg, 2 mg to 200 mg, or 2 mg to 100 mg, or 2 mg to 50 mg, or 4 mg to 25 mg, or 5 mg to 20 mg, or 10 mg to 15 mg, or 50 mg to 200 mg, or 200 mg to 1000 mg, or 1, 2, 3, 4, 5 or more than 5g per dose or composition.

It is to be noted that dosage values may vary with the severity of the condition to be alleviated. For any particular subject, specific dosage regimens may be adjusted over time according to the individual need and the professional judgement of the person administering or supervising the administration of the compositions. Dosage ranges set forth herein are exemplary only and do not limit the dosage ranges that may be selected by medical practitioners. The amount of active compound(s) in the composition may vary according to factors such as the disease state, age, sex, and weight of the individual. Accordingly, in some embodiments, suitable dosages include pediatric dosages or dosages suitable for administration to pregnant females. In some embodiments, suitable dosages include probiotic dosages, such as pediatric probiotic dosages. Dosage regimens may be adjusted to provide the optimum desired response. For example, a single bolus may be administered, several divided doses maybe administered over time or the dose may be proportionally reduced or increased as indicated by the exigencies of the situation. It may be advantageous to formulate parenteral compositions in dosage unit form for ease of administration and uniformity of dosage. The bacterial compositions may be administered daily or more frequently, such as twice or more daily.

The bacterial compositions may be administered prior to, during or after consumption of a food or beverage.

### Detection Methods

Also provided herein are methods of determining the likelihood of development of in a subject of cancer or of a condition associated with IL-17 and/or eosinophils, by determining the levels of one or more bacteria of the genera Prevotella in the subject, and comparing the determined levels to a reference or a healthy individual, such as an individual not diagnosed with cancer or of a condition associated with IL-17 and/or eosinophils, where a reduction or decrease in the levels of one or more bacteria of the genera Prevotella indicates an increased likelihood of development of cancer or of a condition associated with IL-17 and/or eosinophils. In general, a statistically significant difference between the subject and the reference or healthy individual indicates that the subject is likely to develop cancer or of a condition associated with IL-17 and/or eosinophils. In some embodiments, a difference of 1 or 2, on the logarithmic scale, between the subject and the reference or healthy individual may indicate a likelihood of development of cancer or of a condition associated with IL-17 and/or eosinophils in a subject.

In some embodiments, the levels of two or more bacteria of the genera Prevotella in a sample from a subject may be determined.

In some embodiments, determining the likelihood of development of cancer or of a condition associated with IL-17 and/or eosinophils in a subject, include determining the levels of one of more of a metabolite or antigen or products such as IL-17.

By "determining' or "detecting" it is intended to include determining the presence or absence of a substance or quantifying the amount of a substance, such as one or more of the bacteria described herein, or a metabolite as described herein. The term thus refers to the use of the materials, compositions, and methods described herein or known in the art for qualitative and quantitative determinations. An increase or decrease may include a change of any value between 10% and 100%, or of any value between 30% and 60%, or over 100%, for example, a change of about 10%, 20% 30%, 40%, 50%, 60%, 70%, 80%, 90%, 95% or more, when compared to a control. In some embodiments, the increase or decrease may be a change of about 1-fold, 2-fold, 5-fold, 10-fold, 100-fold, or more, when compared to a control.

A subject determined to be likely to develop cancer or a condition associated with IL-17 and/or eosinophils may be treated with a bacterial composition, as described herein.

In some embodiments, the efficacy of the treatment may be monitored by determining the levels of one or more bacteria of the genera Prevotella or part thereof or a metabolite, in a sample from the subject, and comparing the determined levels to previous determinations from the subject.

A "sample" can be any organ, tissue, cell, or cell extract isolated from a subject, such as a sample isolated from a mammal having, suspected of having, or having a predisposition to cancer or of a condition associated with IL-17 and/or eosinophils. For example, a sample can include, without limitation, blood, urine, stool, saliva, or any other specimen, or any extract thereof, obtained from a patient (human or animal), test subject, or experimental animal. A "control" includes a sample obtained for use in determining base-line expression or activity. Accordingly, a control sample maybe obtained from a healthy individual, such as an individual not diagnosed with cancer or of a condition associated with IL-17 and/or eosinophils. A control also includes a previously established standard or reference. Accordingly, any test or assay may be compared with the established standard and it may not be necessary to obtain a control sample for comparison each time. The sample may be analyzed to detect the presence or levels of a Prevotelle, Prevotella gene, genome, polypeptide, nucleic acid molecule, using methods that are known in the art, such as quantitative PCR. The sample may be analyzed to detect the presence or levels of a metabolite or antigen or substance such as IL-17.

In some embodiments, provided herein are methods of modulating an immune response in a subject, the method comprising administering to the subject a composition comprising bacteria from one or more of the families Prevotellaceae, and/or the genera Prevotella. In some embodiments, the modulation of the immune response comprises (or results in) preventing (prophylactically) and/or treating (responsively) cancer or of a condition associated with IL-17 and/or eosinophils. In some embodiments, the composition comprises bacteria from 2 or more (e.g., 2, 3, 4, 5, 6, 7, 8, 9 10, 11, 12, 13, 14, 15, 16, 17, 18, 19, 20, or more, or ranges therebetween) different taxa (e.g., families, genera, species, strains etc.). In some embodiments, the composition comprises at least 104colony forming units (CFU) of bacteria (e.g., at least 1x10⁴CFU, 2x10⁴CFU, 5x10⁴CFU, 1x10⁵CFU, 2x10⁵CFU, 5x10⁵CFU, 1x10⁶CFU, 2x10⁶CFU, 5x10⁶CFU, 1x10⁷CFU, 2x10⁷CFU, 5x10⁷CFU, 1 x10⁸CFU, 2x 10⁸CFU, 5x10⁸CFU, 1x10⁹CFU, 2x10⁹CFU, 5x10⁹CFU, 1x10¹⁰CFU, 2x10¹⁰CFU, 5x10¹⁰CFU, 1x 10¹¹CFU, 2x10¹¹CFU, 5x10¹¹CFU, 1x10¹²CFU, 2x10¹²CFU, 5x10¹²CFU, or more or ranges there between). In some embodiments, the subject has abnormal gut microbiota. In some embodiments, the subject is a human. In some embodiments, the subject is an animal (e.g., livestock, domestic pet, research subject, etc.). In some embodiments, the composition is administered orally, topically, rectally, etc. In some embodiments, the composition is co-administered with one or more additional active agents. In some embodiments, the additional active agents are part of the same formulation. In some embodiments, the additional active agents are administered separately as part of the treatment plan for the prevention and/or control of an infection. In some embodiments, the additional active agent comprises a probiotic component or a prebiotic component. In some embodiments, the additional active agent comprises a small molecule based therapeutic. In some embodiments, the additional small molecule is an antibiotic, a signaling compound, or a compound that attenuates the virulence phenotype of pathogenic microbes. In some embodiments, the additional active agent comprises an antibody or antibody fragment. In some embodiments, the additional active agent comprises a peptide or polypeptide. In some embodiments, the additional active agent comprises a microbe.

In some embodiments, methods described herein comprise assaying the microbiome and/or metabolome of a subject. In some embodiments, assaying the microbiome comprises testing the presence, absence, relative abundance or amount of one or more bacteria in the gut of the subject. In some embodiments, assaying the microbiome comprises testing the presence, absence, relative abundance or amount of one or more bacteria from one or more of the families Prevotellaceae, and/or the genera Prevotella. In some embodiments, assaying the metabolome comprises quantifying amount of one or more metabolites in the gut of the subject. In some embodiments, the assaying is performed on the subject before and/or after administration of the composition.

In some embodiments, provided herein are pharmaceutical compositions comprising bacteria Prevotellaceae, and/or the genera Prevotella, in particular *Prevotella melaninogenica.* In some embodiments, pharmaceutical compositions comprise a therapeutically effective amount of bacteria. In some embodiments, a therapeutically effective amount of bacteria is an amount sufficient to treat or prevent cancer or a condition associated with IL-17 and/or eosinophils in a subject. In some embodiments, a therapeutically effective amount of bacteria is an amount sufficient to activate regulator T cell accumulation in the subject. In some embodiments, pharmaceutical compositions comprise a probiotic or a prebiotic. In some embodiments, the bacteria are alive as vegetative cells and/or spores. In some embodiments, pharmaceutical compositions are formulated for oral, rectal, and/or topical administration. In some embodiments, the pharmaceutical composition is a nutraceutical or a food.

The present invention will be described by means of non-limiting examples referring to the following figures:

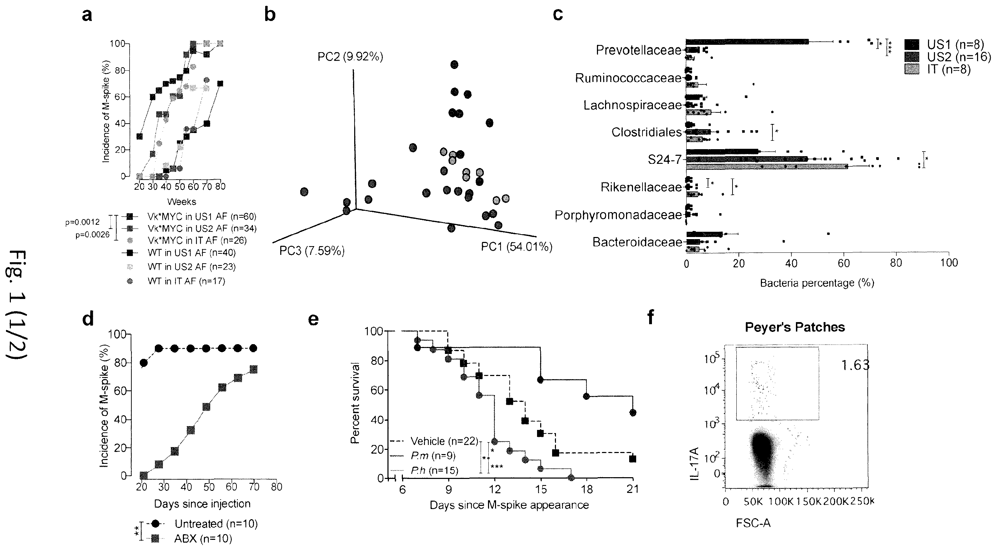

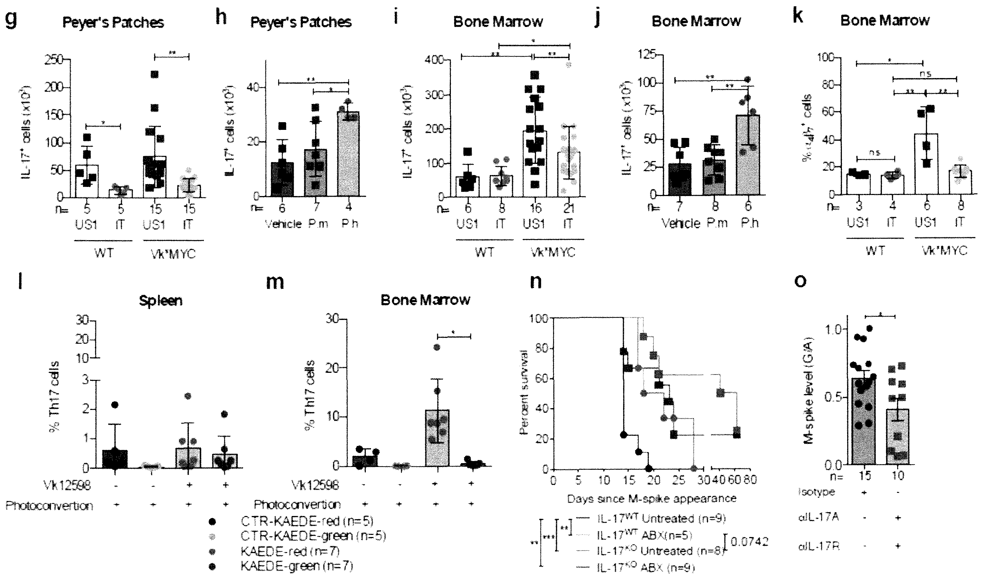

**Fig. 1****. *P. heparinolytica* favors MM progression by promoting BM accrual of IL-17⁺ cells. a M-**spike incidence over time (weeks) in sex-matched Vk*MYC and WT littermates housed in US1, US2 and IT animal facilities (AF) as indicated in the text. Statistical analyses (Two-way Anova). **b** Principal component analysis of fecal microbiota from mice housed in the indicated shelters. **c** Mean ± Standard Error of Mean (SEM) of eight taxa in fecal microbiota between US1 (n=8 of biologically independent mice, US2 (n=16) and IT (n=8) mice relative to total number of reads recovered from each group. Statistical analyses [One-way Anova (Dunn's multiple comparison test)]: **P* <0.05, ***P <0.01, ***P* <0.001. **d** M-spike incidence over time (days) in t-Vk*MYC MM mice either maintained or not under antibiotics. Unpaired t test: Vehicle vs ABX up to 40 days: **P* <0.05. e Overall survival (Kaplan-Meier plot) of t-Vk*MYC MM mice gavaged with vehicle (Vehicle), *P*. *heparinolytica* (P.h) or P melanonogenica (P.m). Long-rank (Mantel-Cox) test: Vehicle vs P.h: *P =* 0.0157; Vehicle vs P.m: *P* = 0.0346, P.m vs P.h: *P* = 0.0002. **f** Representative dot plot of Peyer's Patches IL-17⁺ cells (gated on live cells). **g-j** Number of Peyer's Patches **(g** and **h)** and BM IL-17⁺ cells **(i** and **j)** from mice described in **a (g** and **i)** and **e (h** and **j),** respectively. **k** Quantification of α₄β₇⁺ cells gated on IL-17⁺ cells from the same samples shown in i. Mean ± SD of three independent experiments. Unpaired t test: **P* <0.05; ***P* <0.01. **l-m** Frequency of KAEDE red positive (black and red columns) and negative (gray and green columns) Th17 cells in the spleen **(l)** and BM **(m)** of photoconverted control and diseased Kaede mice. Mean ± SD of three independent experiments. Wilcoxon matched-pairs signed rank test; *P = 0.0156. **n** Survival (Kaplan-Meier plot) of t-Vk*MYC MM mice IL-17 competent (IL-17^{WT}) or deficient (IL-17^{KO}) and maintained or not maintained under antibiotics. Long-rank (Mantel-Cox) test: **P* =0.0332, ***P* =0.0021. **o** M-spike levels are expressed as total gamma globulins/albumin ratio (G/A) in mice within the indicated cohort. Unpaired t test: **P* <0.05. **α-e,g-o** n = number of mice used.

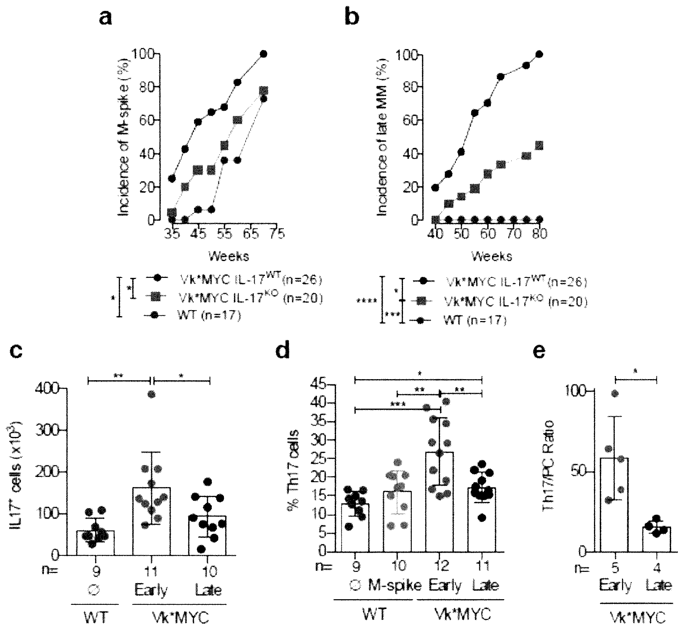

**Fig. 2** **Pro-tumoral role of IL-17 during the early phase of MM. a** M-spike incidence over time (weeks) in cohorts of Vk*MYC mice either competent (Vk*MYC IL-17^{WT}) or deficient for IL-17 (Vk*MYC IL-17^{KO}) and WT littermates. Unpaired t test: **P* <0.05. **b** Incidence of M-spike ≥ 6%, corresponding to symptomatic, Late-MM³³, in the mice depicted in panel **a.** Unpaired t test: **P <0.05; **P* <0.01; ****P* <0.001; *****P* <0.0001. **c and d** Absolute numbers **(c)** and frequency **(d)** of IL-17⁺ cells in the BM of Vk*MYC mice and age-and sex-matched WT littermates. Each dot is representative of an individual mouse. Mean ± SD of five independent experiments. Unpaired t test: **P* <0.05; ***P* <0.01; ****P* <0.001. **e** Ratio between Th17 cells and malignant plasma cells (IRF4/MUM1⁺). Mean ± SD of five independent experiments. Whitney test: **P* <0.0159. **c-e** Specific n values of biologically independent mice are shown.

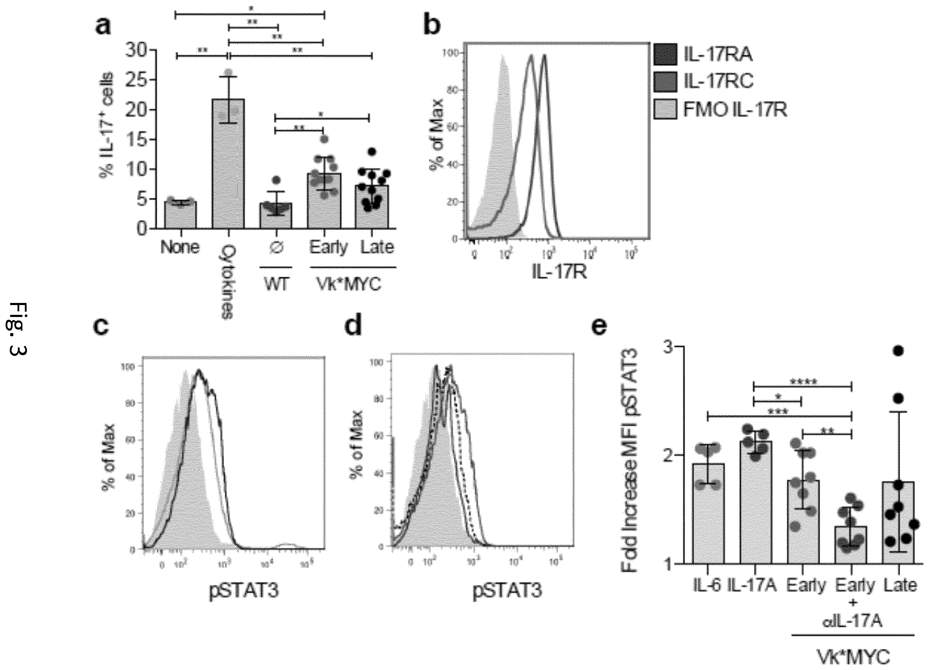

**Fig. 3** **IL-17 promotes STAT-3 phosphorylation in Vk*MYC plasma cells. a** Th17 polarization of OT-II splenocytes cultured for 7 days with BM serum obtained from WT, Early-MM and Late-MM Vk*MYC mice and assessed for intracellular cytokine release by flow cytometry. None and Cytokines refer to the culture condition with or without IL-6, TGF-B1, anti-IL-4 and anti-IFN-γ antibodies, respectively. (None n = 3, Cytokine n=3, WT n=6, Vk*MYC Early n=11, Vk*MYC Late n=11). Mean ± SD of three independent experiments. Unpaired t test: **P* <0.05; ***P* <0.01; ****P* <0.001. **b** Plasma cells were also stained with anti-IL-17RA and anti-IL-17RC antibodies (blue and red line respectively) and analyzed by flow-cytometry; FMO (Fluorescence Minus One) sample was not stained for IL-17R (gray histogram). **c** and **d** Representative histograms and e quantification of Vk*MYC PCs cultured in the presence of either one of the following stimuli: saturating amounts of IL6 (light blue line) or IL-17 (dark blue line), or BM sera from Early- (red line) or Late-MM (black dotted line), or BM sera from Early-MM and anti-IL17 antibodies (purple line). After culture, plasma cells were analyzed by flow-cytometry for STAT3 phosphorylation (pSTAT3). (IL-6 n=5, IL-17A n=5, Vk*MYC Early n=8, Vk*MYC Early + αIL-17A n=8, Vk*MYC Late n=8). Mean ± SD of triplicate independent determinations. Unpaired t test: **P* <0.05; ***P* <0.01; ****P <0.001;* *****P* <0.0001.

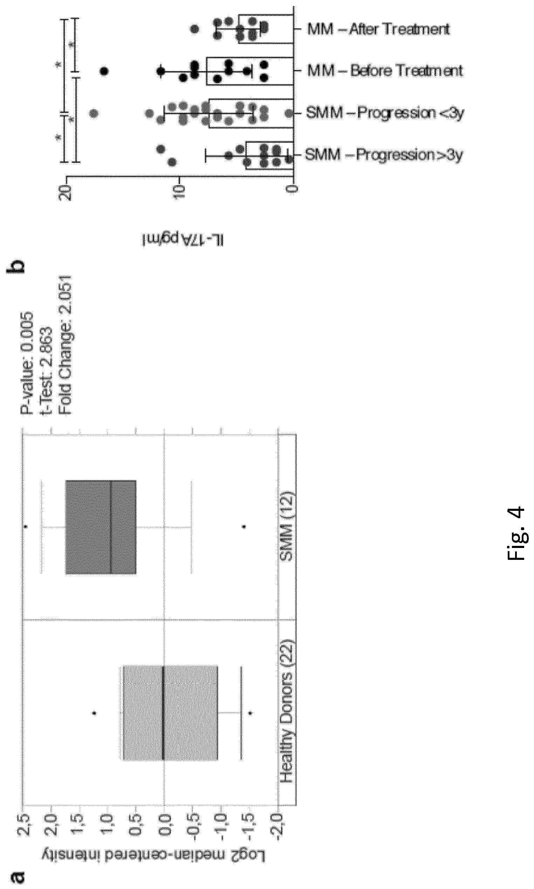

**Fig. 4** **IL-17 levels are increased in the BM of SMM patients rapidly progressing to MM. a** mRNA expression of IL-17RA in primary SMM cells of a cohort of 12 newly-diagnosed patients and 22 matched controls (bone marrow) described in ref. ⁶⁵. The expression pattern for the probe set 205707_at is shown. Statistical analysis (Student t test) is reported. **b** IL-17 levels in the BM plasma of SMM patients that progressed to MM within 3 years since the diagnosis (i.e., < 3 years), or did not progress to MM in the same time frame (i.e., > 3 years). Each dot represents an individual patient. (SMM-Progression >3 n=12, SMM-Progression <3 n=22, MM-Before treatment n=12, MM-After treatment n=11). Data are reported as mean ± SD. Unpaired t test: **P <0.05.*

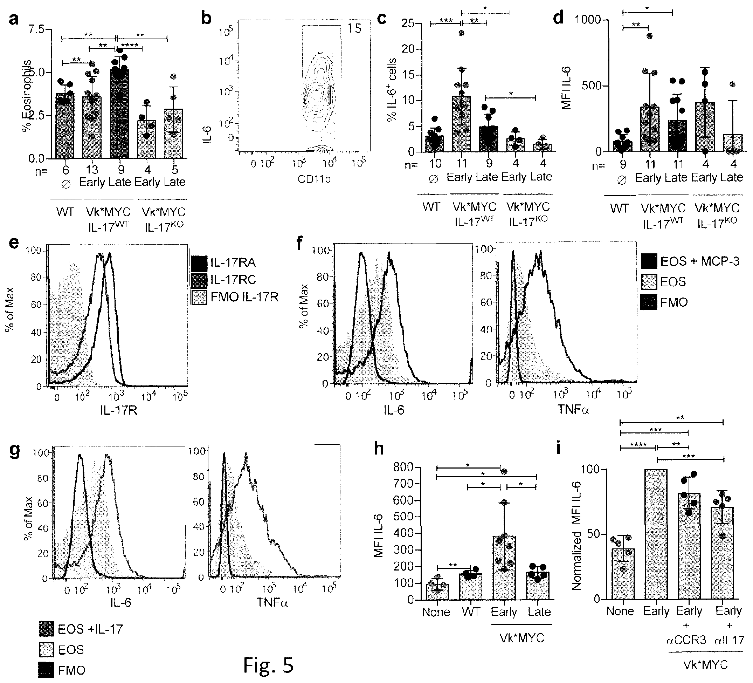

**Fig. 5** **An IL-17-eosinophil axis in the BM of Vk*MYC mice favors disease progression. a** Frequency of BM eosinophils (i.e., CD11b⁺Ly6c^{int}MHC-II⁻Ly6G-SSC^{hi} or CD11b⁺Sig1ec-F⁺ cells) in Vk*MYC IL-17^{WT} and Vk*MYC IL-17^{KO} mice and age- and sex-matched WT littermates. Each dot represents an individual mouse. Mean ± SD of five independent experiments. Unpaired t test: *P *<0.05; **P* <0.01; ****P* <0.001; *****P* <0.0001. **b** Representative dot plot of IL-6⁺ cells (gated on CD11b⁺Sig1ec-F⁺ cells) in the BM. c Percentage of IL-6⁺ cells gated on CD11b⁺Sig1ec-F⁺ cells. Mean ± SD of five independent experiments. Unpaired *t* test. **d** Mean fluorescence intensity (MFI) of IL-6 within Siglec-F⁺CD11b⁺ cells. Mean ± SD of five independent experiments. Unpaired t test. **e** BM derived eosinophils were also stained with anti-IL-17RA and anti-IL-17RC antibodies (blue and red line respectively) and analyzed by flow-cytometry; FMO (Fluorescence Minus One) sample was not stained for IL-17R (gray histogram). **f** Representative histograms of IL-6 and TNF-α production by eosinophils after IL-17A stimulation (red line). FMO samples were not stained for IL-6 or TNF-α. **g** Representative histograms of IL-6 and TNF-α production by eosinophils after MCP-3 stimulation (blue line). FMO samples were not stained for IL-6 or TNF-α. h IL-6 levels (MFI normalized on FMO sample) in eosinophils cultured alone (None; n=4), or in the presence of WT (n=4) or Early-MM (n=8) or Late-MM (n=5) BM serum. Mean ± SD of aggregated data from three independent experiments. Unpaired *t* test. **i** IL-6 levels (MFI normalized on Early-MM sample) in eosinophils cultured alone (None; n=5), or in the presence of Early-MM with or without the addition of anti-CCR3 (n=5) or anti-IL-17A (n=5). Mean ± SD of aggregated data from three independent experiments. Paired t test. a, c-d Specific n values of biologically independent mice are shown.

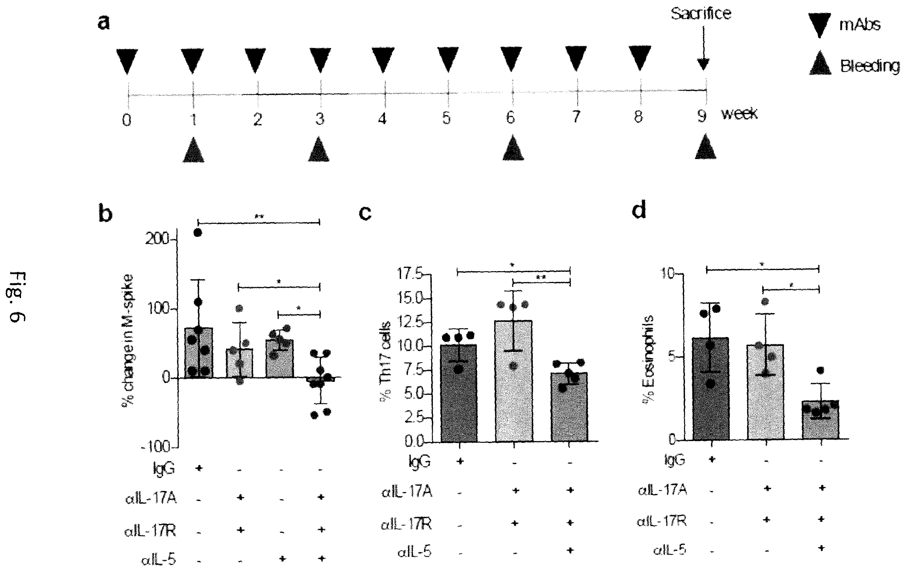

**Fig. 6** **IL-17-eosinophil axis neutralization delays disease progression in Vk*MYC mice. a** Schematic representation of the experiment. **b** Percentage change in M-spike in mice within the indicated cohort (Isotype and αIL-17A, αIL-17R, αIL-5: n=8 mice/group, αIL-17A, αIL-17R and αIL-5: n=5 mice/group) during the observation period. Frequency of BM Th17 (i.e., CD3⁺CD4⁺IL-17⁺) cells c and eosinophils (i.e., CD11b⁺Ly6c^{int}MHC-II⁻Ly6G-SSC^{hi} **d)** was assessed by flow cytometry. Each dot represents an individual mouse. c Mean ± SD of two independent experiment. (Isotype n=4, αIL-17A, αIL-17R n=4, αIL-17A, αIL-17R, αIL-5 n=5). Unpaired t test: **P <0.05; **P* <0.01. **d** Mean ± SD of two independent experiment. (Isotype n=4, αIL-17A, αIL-17R n=4, αIL-17A, αIL-17R, αIL-5 n=5). One-way Anova *P* = 0.0101.

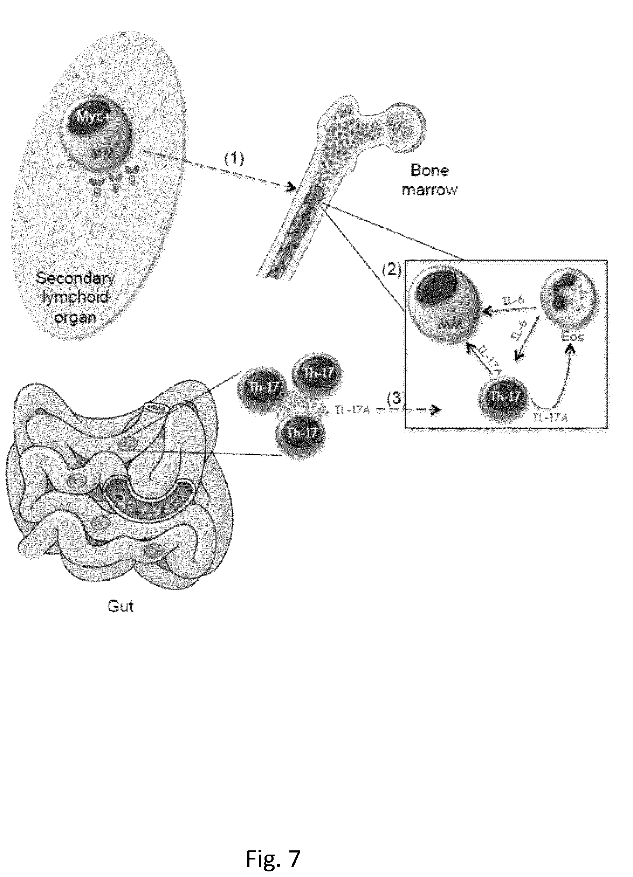

**Fig. 7** **IL-17-producing cells indeuced by gut microbiota favor MM progression.** (1) Upon AID-dependent MYC activation in germinal centers, a B cell stochastically acquires the characteristics of malignant plasma cell (MM) and migrates to the BM. (2) Within the BM niche, a favorable cytokine milieu induces Th17 skew and eosinophil (Eos) activation, thus establishing a positive-feedback loop that is self-amplifying and sustains MM progression. (3) A selected gut microbiota locally favors the expansion of Th17 cells, which migrate to the BM niche, where they further contribute to the eosinophi1-Th17-MM cells network.

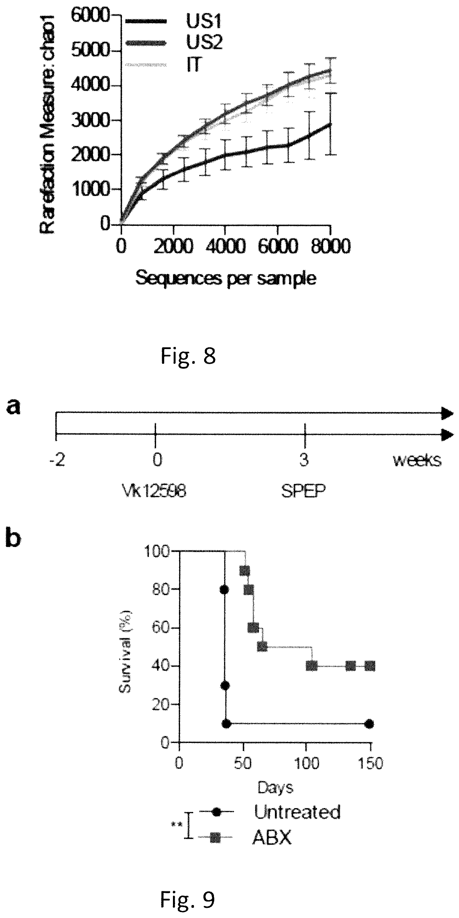

**Fig. 8** **Microbiome analyses of stool samples from mice housed in the different animals' facilities.** CHAO1 index analysis of fecal microbiota from Vk*MYC and WT littermates housed in US1 (n=8 of biologically independent mice), US2 (n=16) and IT (n=8) animal facilities.
**Fig. 9** **Antibiotic treatment improves the overall survival in t-Vk*MYC MM mice.** a Schematic representation of the experiment reported in Fig. 1d. Mice were monitored for M-spike appearance as described in the Methods section. **b** Survival (Kaplan-Meier plot) of t-Vk*MYC MM mice maintained or not under antibiotic in drinking water (n=10/group) is reported. Long-rank (Mantel-Cox) test: **P* =0.0027

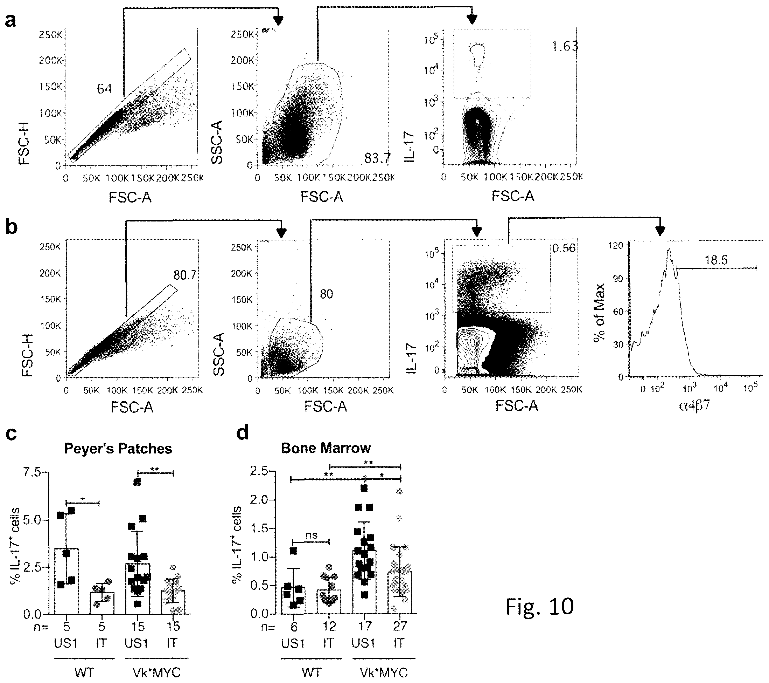

**Fig. 10** **IL-17⁺ cells are enriched in the Peyer's patches and BM of Vk*MYC mice housed in the US1 animal facility.** Gating strategy relative to IL-17⁺ cells in the Peyer's Patches **(a)** for the data reported in Fig. 1f, g, h and Fig 9c, and in the BM **(b)** for the data reported in Fig. 1i, j, k, Fig. 2c, d, Fig. 6c, Fig 9d, Fig. 12a, b, c, d and Fig. 17b. Frequency of IL-17⁺ cells in the Peyer's Patches (c) and BM **(d)** from Vk*MYC mice and sex- and age-matched WT littermates housed in US1 or in IT AF. Mean ± SD of three independent experiments. Unpaired *t* test: **P* <0.05; ***P* <0.01; ****P* <0.001.

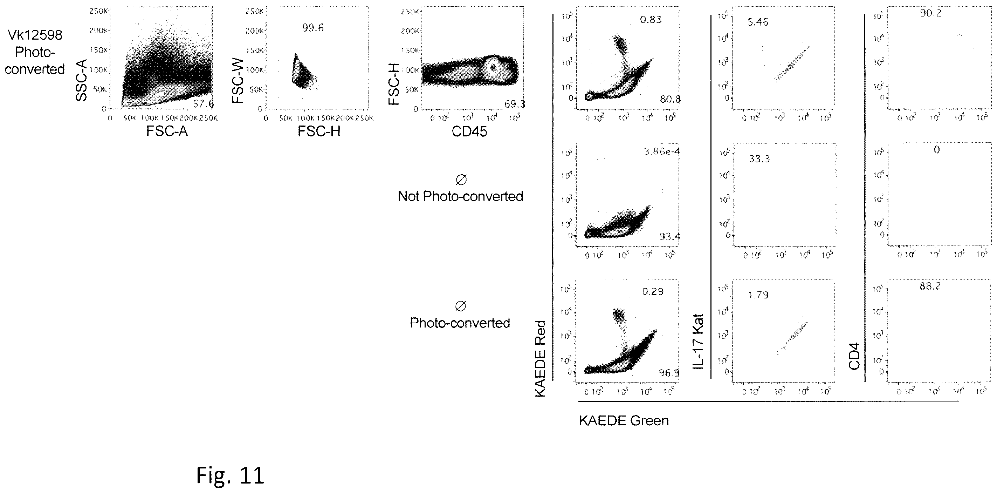

**Fig. 11** **Gating strategy relative to the quantification of gut-experienced Th17 cells in photoconverted Kaede mice.** Representative dot plots of BM cells from treated, photoconverted Kaede mice, untreated, not photoconverted Kaede mice and untreated, photoconverted Kaede mice (Fig. 11 and m). Right panels show frequency of IL-17A FP635⁺ cells within the KAEDE red positive and KAEDE red negative CD4⁺ T cells.

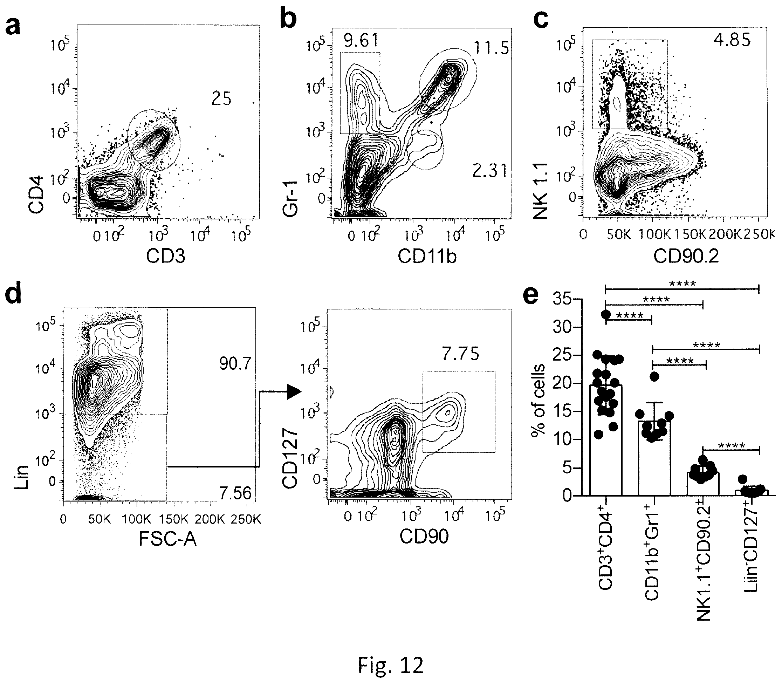

**Fig. 12** **Characterization of IL-17⁺ cells in the BM of Vk*MYC and WT mice.** Representative plots of CD3⁺CD4⁺ **(a),** CD11b⁺Gr-1⁺ **(b),** NK1.1⁺CD90.2⁻ (c) and CD90⁺CD127⁺ cells **(d)** pre-gated on Lin- cells (lower left panel), all gated on IL-17⁺ cells, in the BM of SPF Vk*MYC mice. Dead cells were excluded by live/dead staining. e Panels report the percentage of the different subsets. (CD3⁺CD4⁺ n=18; CD11b⁺Gr-1⁺ n=9; NK1.1⁺CD90.2⁻ n=9; Lin⁻ CD127⁺ n=9). Mean ± SD of three independent experiments. One-way Anova P < 0.0001. Unpaired t test: **P* <0.05; ***P* <0.01; ****P* <0.001; *****P* <0.0001.

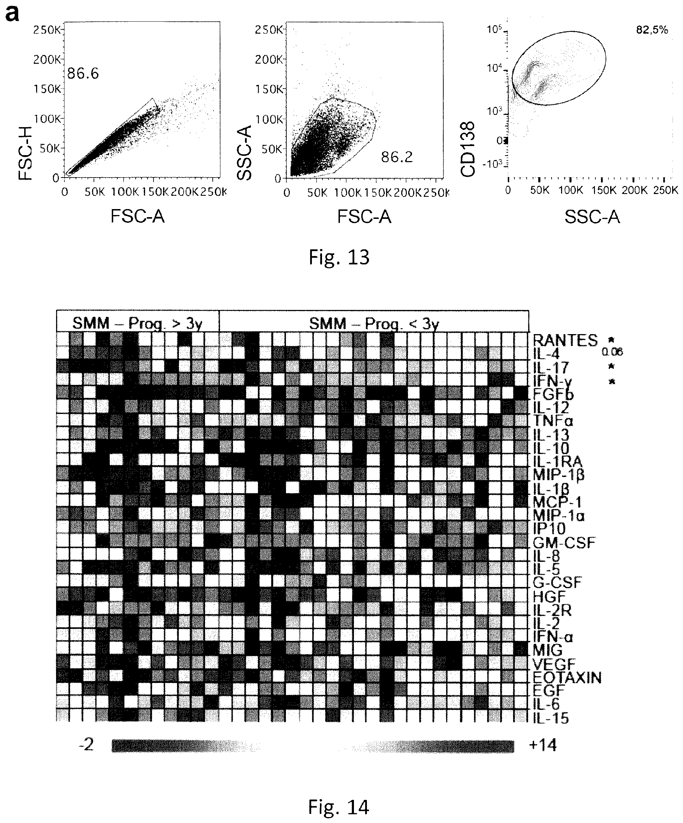

**Fig. 13** **Gating strategy relative to BM-derived CD138⁺ plasma cells.** Gating strategy for the flow-cytometry analyses of CD138⁺ plasma cells obtained from the BM of Vk*MYC mice, and reported in Fig. 3b, c, d.
**Fig. 14** **Quantification of cytokines and chemokines in the BM serum obtained from SMM patients.** Cytokines and chemokines were quantified in the BM sera of SMM patients that progress to MM < 3 years, or > 3 years as described in the Materials and Methods section. The Heat map reports the relative expression (log base 2) of the indicated BM soluble factors ranked based on their differential expression between the two groups.

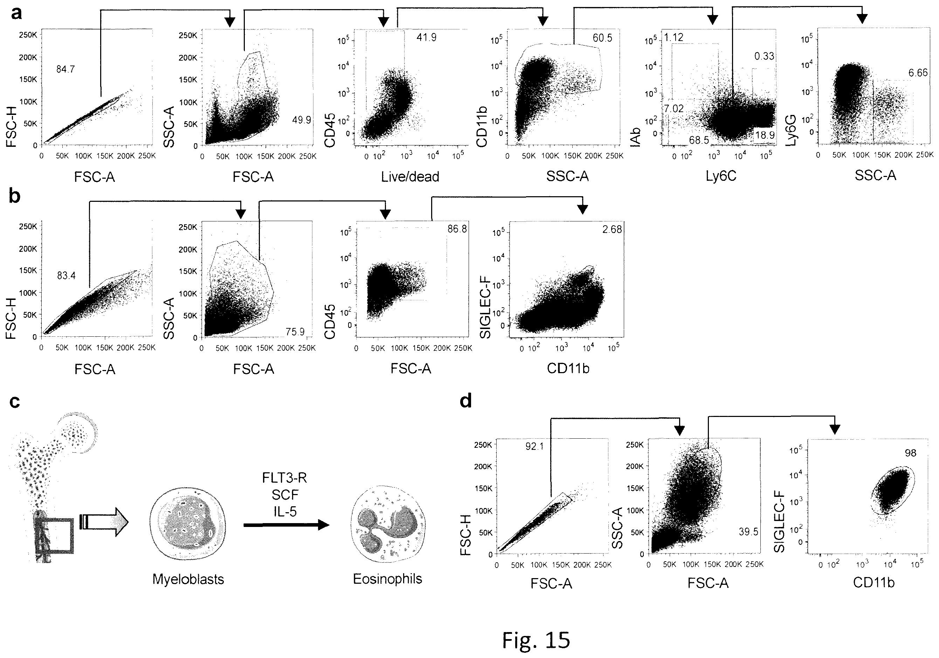

**Fig. 15** **Quantification of BM eosinophils and their *in vitro* differentiation. a** Gating strategy relative to the quantification of the frequency of eosinophils in the BM of Vk*MYC, Vk*MYC IL-17ko and t-Vk*MYC MM bearing mice in Fig. 5a and 6d. **b** Gating strategy relative to the quantification of IL-6-producing eosinophils from the BM of Vk*MYC, Vk*MYC IL-17ko and t-Vk*MYC MM bearing mice in Figs. 5b, c and d. c Graphic representation of the *in vitro* differentiation of BM derived eosinophils, (see Materials and Methods section for further information). **d** Representative gating strategy of BM derived eosinophils after 12 days of culture with the described cytokines, relative to data reported in Figs. 5e, f, g, h, and i.

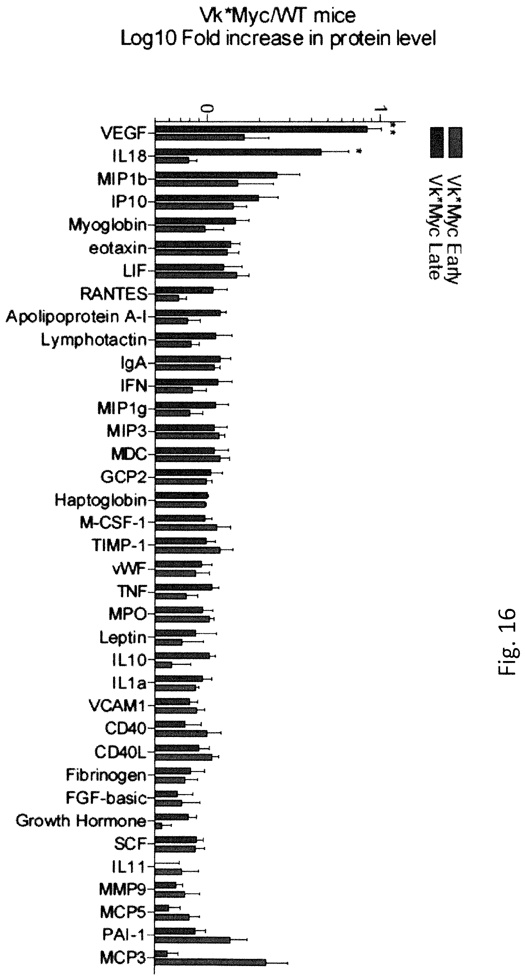

**Fig. 16** **Quantification of cytokines and chemokines in the BM of Vk*MYC mice.** Cytokines and chemokines were quantified in the BM of sex- and age-matched Early-MM (n=7), Late-MM Vk*MYC (n=5) and WT mice (n=5). The graph reports the relative amount (log base 10) of the indicated soluble factors normalized to the values obtained in the BM of WT mice. Unpaired t-test *P<0.05;**P<0.002.

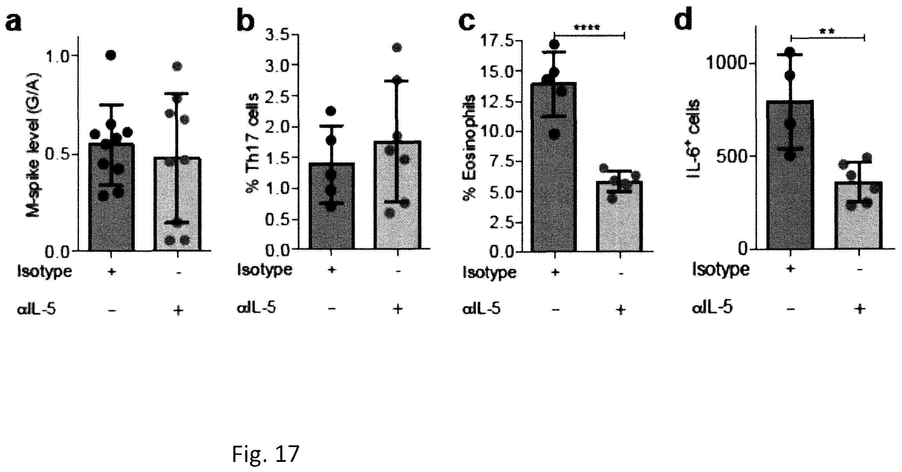

**Fig. 17** **Treatment with anti-IL-5 antibodies does not impact disease progression in t-Vk*MYC MM mice.** As indicated in the Material and Methods section, t-Vk*MYC MM mice were treated with anti-IL-5 antibodies (αIL-5) and euthanized for M-spike detection and BM analysis five weeks later. **a** M-spike levels are expressed as total gamma globulins/albumin ratio (G/A) in t-Vk*MYC MM mice within the indicated cohort at the time of sacrifice (Isotype n=10, αIL-5 n=9). **b** Frequency of Th17 cells in the indicated cohort (Isotype n=5, αIL-5 n=7). c Frequency of eosinophils in the indicated cohort (Isotype n=5, αIL-5 n=6). Each dot represents an individual mouse. Data are reported as mean ± SD. Unpaired t test: ****P <0.0001. **d** Number of IL-6⁺ eosinophils in the indicated cohort (Isotype n=5, αIL-5 n=6). Unpaired t test: ***P <0.01.*

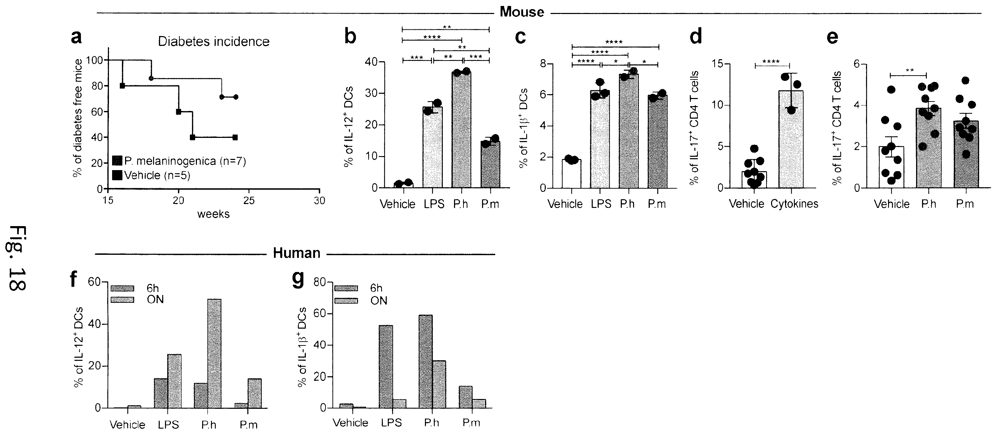

**Fig 18****. a. Kaplan Meier plot of incidence of diabetes in 6-week-old NOD mice** treated with Ampicillin (1 g/L), Vancomycin (0.5 g/L), and Neomycin (1 g/L) in the drinking water and Metronidazole (2 mg/mouse) by oral gavage 3 times per week. After two weeks of antibiotic treatment, mice received 10⁹ CFU of *Prevotella melaninogenica* (blue line) or PBS (Vehicle) trice a week until the end of the experiment. Mice were also bled weekly for the monitoring of glycemia. Animals were considered diabetic when glycemia exceed 250 mg/dl for two consecutive weeks. Log-rank test (Mantel-Cox), P= 0,2436. b-c, Bone marrow cells from one 8-week-old C57BL/6 mouse were cultured in IMDM 10% FBS in the presence of 25 ng/ml of recombinant murine GM-CSF and 5 ng/ml of recombinant murine IL-4 to generate DCs. At day 6, DC maturation was induced by overnight culture with 1ug/m1 LPS from E. coli, or 1mg/ml heat killed *Prevotella heparinolytica* (*P.h*) or *Prevotella melaninogenica (P.m).* Vehicle: fresh medium. The next day, DCs were assessed for intracellular IL-12 (b) and IL-1β (c) production by flow cytometry. Mean ± SD of one experiment representative of three. Statistical analysis: One-way ANOVA: * P<0.05, ** P < 0.01, *** P < 0.001, **** P < 0.0001. d-e, Splenocytes from C57BL/6 mice were cultured in IMDM, 10% FBS in the presence of αCD3 and αCD28 Dynabeads, and either IL-6 (20ng/ml), TGFβ (2ng/ml), αIFNγ (10ug/ml) and αIL-4 (10ug/ml; cytokines) to induce Th17 polarization (d), or 100ug/ml *Ph* or *Pm* (e). After 7 days, cells were stimulated for 6h with PMA-IONO (the last 5h in the presence of BFA), stained for CD3, CD4, CD8 and IL-17, and analyzed by flow cytometry. Mean ± SEM of tree independent experiments. Statistical analysis: Student t-test: **** P < 0.0001 (d) and One-way ANOVA: ** P < 0.01 (e). f-g. CD14⁺ monocytes were isolated by magnetic sorting from PBMCs from a healthy donor and cultured in RPMI 10% FBS in the presence of 170 ng/ml of recombinant human GM-CSF and 24 ng/ml of recombinant human IL-4 to generate DCs. At day 6, DC maturation was induced either by overnight (ON) or 6 hours (6h) culture with 100ng/ml LPS from E. coli, or 1mg/ml of heat killed *Ph* or *Pm.* Vehicle: fresh medium. DCs were assessed for intracellular IL-12 (f) and IL-1β (g) production by flow cytometry. Data are reported as the mean of a single determination.

### DETAILED DESCRIPTION OF THE INVENTION

### Examples

### MATERIALS AND METHODS

**Patients and BM plasma samples.** Bone marrow (BM) plasma aspirates were obtained from patients fulfilling the International Myeloma Working Group (IMWG) diagnostic criteria after informed written consent, in compliance with all the relevant ethical regulations, and with full ethical approval from the Mayo Clinic institutional review board (authorization #12-001145).

Patient's disease staging, collection sample date and MM diagnosis date are reported in Table 1.

**Table 1. Patients' information**

| **Pts** | **DX at Collection Sample** | **Collection Sample Date** | **MM DX Date** | **Group** |
|---|---|---|---|---|
| 1 | SMM | 3-Aug-04 | 25-Jun-10 | Progression >3 years |
| 2 | SMM | 11-Aug-05 | 25-Jun-10 | Progression >3 years |
| 3 | SMM | 28-Aug-06 | 25-Jun-10 | Progression >3 years |
| 4 | SMM | 20-Jul-07 | 24-Apr-14 | Progression >3 years |
| 5 | SMM | 23-Jul-09 | 24-Apr-14 | Progression >3 years |
| 6 | SMM | 8-Oct-07 | 6-Apr-11 | Progression >3 years |
| 7 | SMM | 27-Jan-06 | 10-Mar-10 | Progression >3 years |
| 8 | SMM | 29-Mar-05 | 14-Sep-10 | Progression >3 years |
| 9 | SMM | 13-Nov-06 | 4-Sep-15 | Progression >3 years |
| 10 | SMM | 24-Oct-05 | 10-Sep-10 | Progression >3 years |
| 11 | SMM | 18-Apr-06 | 10-Sep-10 | Progression >3 years |
| 12 | SMM | 28-Jun-06 | 1-Jun-10 | Progression >3 years |
| 13 | SMM | 16-Aug-07 | 25-Jun-10 | Progression < 3 years |
| 14 | SMM | 25-Sep-08 | 25-Jun-10 | Progression < 3 years |
| 15 | SMM | 19-May-06 | 24-Nov-08 | Progression < 3 years |
| 16 | SMM | 21-Aug-07 | 24-Nov-08 | Progression < 3 years |
| 17 | SMM | 3-Oct-08 | 4-Dec-09 | Progression < 3 years |
| 18 | SMM | 13-Dec-05 | 12-Mar-07 | Progression < 3 years |
| 19 | SMM | 7-Oct-08 | 14-Oct-09 | Progression < 3 years |
| 20 | SMM | 30-Jun-11 | 11-Jan-12 | Progression < 3 years |
| 21 | SMM | 26-Sep-07 | 27-Aug-08 | Progression < 3 years |
| 22 | SMM | 27-Nov-12 | 2-Mar-13 | Progression < 3 years |
| 23 | SMM | 2-Dec-10 | 6-Apr-11 | Progression < 3 years |
| 24 | SMM | 9-Jun-10 | 24-May-11 | Progression < 3 years |
| 25 | SMM | 2-Sep-08 | 10-Mar-10 | Progression < 3 years |
| 26 | SMM | 19-Jun-09 | 20-Jul-10 | Progression < 3 years |
| 27 | MGUS | 2-Feb-11 | 6-Feb-13 | Progression < 3 years |
| 28 | SMM | 3-Mar-09 | 29-Sep-09 | Progression < 3 years |
| 29 | SMM | 10-Oct-06 | 7-Jul-08 | Progression < 3 years |
| 30 | SMM | 15-Aug-05 | 31-Jul-07 | Progression < 3 years |
| 31 | SMM | 30-Nov-04 | 13-Apr-05 | Progression < 3 years |
| 32 | SMM | 18-Oct-07 | 16-Sep-08 | Progression < 3 years |
| 33 | SMM | 23-May-05 | 1-Dec-05 | Progression < 3 years |
| 34 | SMM | 24-Apr-07 | 8-Feb-10 | Progression < 3 years |
| 35 | MGUS | 31-Jul-09 | 19-Feb-10 | Progression < 3 years |
| 36 | SMM | 12-Jun-09 | 23-Dec-09 | Progression < 3 years |
| 37 | MM | 4-Dec-09 | 4-Dec-09 | Before Treatment |
| 38 | MM | 14-Oct-09 | 14-Oct-09 | Before Treatment |
| 39 | MM | 28-Apr-10 | 28-Apr-10 | Before Treatment |
| 40 | MM | 27-Aug-08 | 27-Aug-08 | Before Treatment |
| 41 | MM | 4-Apr-11 | 6-Apr-11 | Before Treatment |
| 42 | MM | 24-May-11 | 24-May-11 | Before Treatment |
| 43 | MM | 14-Sep-10 | 14-Sep-10 | Before Treatment |
| 44 | MM | 11-Mar-08 | 11-Mar-08 | Before Treatment |
| 45 | MM | 20-Jul-10 | 20-Jul-10 | Before Treatment |
| 46 | MM | 6-Feb-13 | 6-Feb-13 | Before Treatment |
| 47 | MM | 7-Jul-08 | 7-Jul-08 | Before Treatment |
| 48 | MM | 20-Sep-10 | 10-Sep-10 | Before Treatment |
| 49 | MM | 9-Feb-10 | 8-Feb-10 | Before Treatment |
| 50 | MM | 7-Aug-09 | 24-Nov-08 | After Treatment |
| 51 | MM | 12-Nov-07 | 12-Mar-07 | After Treatment |
| 52 | MM | 23-Sep-14 | 24-Apr-14 | After Treatment |
| 53 | MM | 18-May-12 | 11-Jan-12 | After Treatment |
| 54 | MM | 3-Jun-14 | 11-Jan-12 | After Treatment |
| 55 | MM | 3-Jan-12 | 6-Apr-11 | After Treatment |
| 56 | MM | 12-Jun-12 | 24-May-11 | After Treatment |
| 57 | MM | 16-Feb-15 | 24-May-11 | After Treatment |
| 58 | MM | 10-Nov-05 | 13-Apr-05 | After Treatment |
| 59 | MM | 14-Mar-11 | 10-Sep-10 | After Treatment |
| 60 | MM | 21-Mar-12 | 8-Feb-10 | After Treatment |

| | | | | |
|---|---|---|---|---|
| Abbreviations: Pts, patients; DX, Diagnosis. | | | | |

BM plasma samples were obtained by centrifugation of BM aspirates and cryopreserved in the gas-phase of liquid nitrogen
**IL-17 quantification in human BM plasma.** BM plasma samples from the Mayo Clinic Rochester biobank were analyzed with Cytokine Human Magnetic 30-Plex panel for Luminex platform (LHC6003M, Life Technologies, Waltham, MA) and acquired on a Luminex 200 system equipped with with xPONENT 3.1 software (Thermo Fisher Scientific, Waltham, MA).

**Mice.** All mice used in this study were on a C57BL/6 genetic background. WT C57BL/6J mice were purchased from Charles River Breeding Laboratories, Calco IT, or The Jackson Laboratories, Bar Harbor, ME. In Vk*MYC transgenic mice¹⁴ the activation of the transcription factor MYC, whose locus is found rearranged in half human MM tumors⁶² including SMM⁶³, occurs sporadically through the exploitation of the physiological somatic hypermutation process in germinal center B cells. Within a year, although with variable intensity, all mice develop a monoclonal plasmacytosis confined to the BM, a measurable serum M-spike, and progressively show typical endorgan damage¹⁴. The model has been already validated as a faithful model to predict single agent drug activity in human MM^{14,17}. IL-17^{KO} mice²⁹ were kindly provided by Dr. Yoichiro Iwakura (Institute of Medical Science, Tokio, Japan). To avoid genetic drifting, Vk*MYC mice were backcrossed into IL-17^{KO} mice for at least 6 generations before generating homozygous Vk*MYC IL-17^{KO} breeding pairs. Vk*MYC mice were screened by Real Time PCR in order to identify experimental Vk*MYC^{+/-} animals with the following primers: primer 1 (5'-ACAGCTACGGAACTCTTGTGCGT-3' SEQ ID No. 1), primer 2 (5'-TCAGCCAAGGTTGTGAGGTTGCA-3' SEQ ID No. 2). C57BL/6-Tg(TcraTcrb) 1100Mjb/J (OTII) mice³⁴ were originally provided by Dr. William R. Heath (University of Melbourne, Parkville, Victoria, Australia). Kaede-transgenic mice on a C57BL/6 background were generated by Dr. Miwa Yoshihiro at the University of Tsukuba, Japan²⁶. All these mice were maintained under specific pathogen-free conditions (i.e. the rodents were housed in isolated rooms, fed sterilized food and water, and routinely tested and determined free of designated pathogens capable of interfering with research objectives; SPF) in the San Raffaele facility and experiments were performed according to state guidelines and approved by the European Community Guidelines (Authorizations #574, #1147, #863). KAEDE-transgenic mice were crossed with IL-17A FP635 reporter knock in mice²⁷, all on the C57BL/6 background. For photoconversion, the small intestine of anesthetized Kaede/FP635-transgenic mice was subjected to lighting using a Blue Wave LED Prime UVA (Dymax), essentially as described before²⁸. Control mice were sham operated. These animals were maintained under SPF conditions in the Universititsklinikum Hamburg-Eppendorf facility and treated in accordance with the European Community Guidelines and with the approval of the Universitätsklinikum Hamburg-Eppendorf Institutional Animal Care and Use Committee (authorization # 62/14). The animals reported in Figure 1a, b and c were bred and maintained in a conventional animal facility (i.e. the rodents were housed in dedicated rooms, and routinely tested for designated pathogens; US1 and US2) at he Mayo Clinic Arizona, under The Mayo Foundation Institutional and Albert Einstein College of Medicine Animal Care and Use Committee approval #A01948. US1 colony belongs to animals generated before 2014 from breeding between littermate Vk*MYC mice. US2 colony belongs to animals generated after 2015 from breeding between Vk*MYC homozygous males and C57BL/6J females purchased from the Jackson lab. Animals within the IT colony were rederived into C57BL/6J mice from the Charles Rivers, and generated in the period 2012-2018 from breeding between Vk*MYC homozygous mice and WT littermates. When appropriate, animal diet was specified in the Results section. Animal facilities were constantly monitored for the presence of relevant pathogens and resulted free of those pathogens.

**Serum Protein Electrophoresis.** Mouse blood was periodically collected in Eppendorf by retro-orbital sampling. Semi-automated electrophoresis was performed on the Hydrasys instrument (Sebia, Lissex, France). According to the manufacturer's instructions, 10 µL of undiluted serum were manually applied to the Hydragel agarose gels (Sebia). The subsequent steps: electrophoresis (pH 9.2, 20W constant current at 20°C), drying, amidoblack staining, de-staining and final drying were carried out automatically. The use of Hydrasys densitometer and Phoresis software (Sebia) for scanning resulting profiles provided accurate relative concentrations (percentage) of individual protein zones. M-spike levels were calculated as total gamma globulins/albumin ratio (G/A)¹⁷. **Microbiome Analysis.** Bacterial DNA from 50 mg of fecal material was extracted using PowerFecal DNA Isolation Kit (MoBio) following manufacturer's instruction with only one minor modification in lyses time (15 min instead of 5 min) to try to retrieve all difficult-to-lyse bacteria. Purified DNA was quantified and 200 ng per reaction were used to amplify 16S V3-5 regions using barcoded sample-specific primers and FastStart High Fidelity System (Roche) with this thermocycler program: 95°C for 5 min, 40 cycles of (95°C for 30", 55°C for 45" and 72°C for 1 min) and stored at 4°C until usage. Amplicons were loaded on 1% agarose gel and purified with QiaQuick Gel Extraction kit (Qiagen) and AMPure XP beads (Beckman Coulter) to remove primer dimers and used for emulsion-PCR following 454 GS Junior manufacturer's instruction (Roche). Then, emulsion-PCR was purified and captured beads with inventors' correct amplicons were used to load the instruments for the sequencing run. After quality filtering, resulting sequences (>250bp) were analyzed with QIIME software (1.6.0). Principal component analysis (PCA) was performed on the resulting matrix of unweighted UniFrac distances between samples and statistical analysis was performed on the proportional representation of taxa (summarized to Phyla, Class, Order, Family and Genus levels), using unpaired Student's t-tests.

**Antibiotic treatment and challenge with tumor cells.** Two weeks before I.V. tumor cell challenge [1 × 10⁶ Vk12598 cells derived from a MM Vk*MYC mouse ¹⁴], Ciprofloxacin (300 mg/L) and Metronidazole (1g/L; Sigma-Aldrich), known to eliminate the majority of intestinal bacteria⁶⁴, were added to the drinking water of 8-10 week old WT or IL-17^{KO} C57BL/6J recipients, and mice were maintained on antibiotics throughout the duration of the experiment. Mice were monitored for M-spike appearance as described above and sacrificed within 70 days. Vk12598 cells were generated in Bergsagel lab and were not authenticated. As these cells do not grow *in vitro,* they were not tested for mycoplasma contamination.

**Bacteria cultivation and mice infection.** *P. heparinolytica* DSM 23917 and *P. melaninogenica* DSM 7089 (DSMZ, Germany) were cultured in Brain Heart Infusion (BHI) medium at 37°C under anaerobic conditions, following manufacturer's instructions. 50 µl of bacterial growth where then transferred on chocolate agar plates and cultivated at 37°C in AnaeroJar 2.5L Jar System (OXOID) and using AnaeroGen 2.5L (Thermo Scientific) in order to generate an anaerobic atmosphere. To infect mice with the selected *Prevotella* strains, Ampicillin (1g/L; Sigma-Aldrich), Vancomycin (0,5 g/L; Sigma-Aldrich), and Neomycin (1g/L; Sigma-Aldrich) were added to the drinking water of 6 week old WT C57BL/6J and Metronidazole (2mg/mouse; Sigma-Aldrich) was administered by oral gavage 3 times per week. Two week later, antibiotic-treated animals were infected by gavage with with *P. heparinolytica* or *P. melaninogenica* for 3 consecutive days/week, until the end of the experiment. Each recipient mouse received an oral gavage of 200 µl. *Prevotella* in the stool of infected mice was confirmed by RT-PCR. After two weeks of bacteria infection, mice were challenged I.V. with 1×10⁶ Vk12598 cells. For disease monitoring, mouse blood was collected by retro-orbital sampling once a week starting from the third week since tumor challenge and analyzed by Serum Protein Electrophoresis as described above.

**Antibody treatments.** αIL-5 (Clone TRFK5, BioxCell), or αIL-17A (Clone P59234.19, Amgen) and αIL-17R (Clone PL-31280, Amgen) or αIL-17A, αIL-17R and αIL-5, or isotype control (GL117, rat IgG2a) were injected i.p. (once a week for 9 weeks, 150 µg of each monoclonal antibody per mouse) in Early-MM Vk*MYC mice. Every three weeks mice were bled for M-spike quantification. Five days after the last injection mice were sacrificed and their BM assessed for the presence of Th17 cells and eosinophils. In experiments with the Vk12598 murine cell line, sex- and age-matched C57BL6J or IL-17^{KO} mice were challenged i.v. with 1 × 10⁶ Vk12598 cells, and C57BL6J mice were weekly injected i.p. with 100 µg per mouse of αIL-5 (Clone TRFK5, BioxCell), or αIL-17A (Clone P59234.19, Amgen) and αIL-17R (Clone PL-31280, Amgen), their combination, or isotype control (GL117, rat IgG2a) starting from the week of tumor challenge. Every week mice were bled for M-spike quantification.

**Collection of BM serum and cells.** Each femur devoid of epiphyses was placed into a 0.5 ml eppendorf tube whose bottom was pierced with a 16G needle. The pierced eppendorf tube containing the bone was subsequently placed into a 1.5 ml eppendorf tube and centrifuged (Heraeus^{™} Pico^{™} 17 Microcentrifuge, ThermoFisher Scientific, Waltham, MA USA) for few seconds. The BM pelleted material, containing both serum (approximately 10 µl) and cells was resuspended in 100 µl PBS, and used for flow cytometry analyses. Alternatively, the resuspended material was centrifuged again to separate diluted serum from cells, and stored at -80°C.

**Flow Cytometry.** Peyer's Patches were removed from the Small Intestine, and gently disaggregated with the help of tweezers. BM cells from the same animals were collected as described above. Single cell suspensions were labeled with fluorochrome-conjugated monoclonal antibodies (either from BD Bioscience, Buccinasco IT, Biolegend Europe, Uithoorn The Netherlands, or eBioscience Inc, Prodotti Gianni, Milan, IT, or R&D Systems, Space Import-Export srl, Milano, Italy) after neutralization of unspecific binding with FcR blocker (BD Biosciences), and acquired by BD LSR Fortessa^{™}(BD Biosciences). The antibodies used were: αIL17A (clone TC11-18H10, cat 559502), αIL17RA (clone PAJ-17R cat 17-7182-80), α α4B7 (clone DATK32, cat 120607), αCD3 (clone 145-2C11, cat 100330), αCD8 (clone 53-6.7, cat 560776), αCD4 (clone GK1.5, cat 100536), αNK1.1 (clone PK136, cat 108705), αCD90.2 (clone 30-H12, cat 105324), αCD138 (clone 281-2, cat 553714), αpSTAT3 (clone pY705, cat 557815), αIL6 (clone MP5-20F3, cat 561367), αSiglecF (clone E50-2440, cat 562068), αCD45 (clone 30-F11, cat 561487), αLy-6G (clone 1A8, cat 561236), αLy6C (clone HK1.4, cat 128017), αCD11b (clone M1/70, cat 101224), αCD127 (clone A019D5, cat 351303), Lin (clone 17A2/RB6-8C5/RA3-6B2/Ter-119/M1/70, cat 133301), αCD11c (clone N418, cat 117318), αI-Ab (clone 25-9-17, cat 114406), either from BD Bioscience, Biolegend Europe, Uithoorn The Netherlands, or eBioscience Inc, Prodotti Gianni, Milan, IT, polyclonal αIL17RC (cat FAB2270A) from R&D Systems, Space Import-Export srl, Milano, Italy). For surface staining all antibodies were diluted 1:200, with the exception of αIL17RC diluted 1:20, for intracellular staining antibodies were diluted 1:100. Data were analyzed using the FlowJo software (TreeStar Inc, Ashland, OR, USA). Cells were also assessed for intracellular cytokine production after 6 hours at 37°C of stimulation with Phorbol Myristate Acetate (PMA)/ionomycin. GolgiPlug^{®} (BD Bioscience) was added to the samples during the last 5 hours of culture. After incubation, cells were washed and stained for surface markers 15 minutes at 4°C, fixed and permeabilized with Fixation/Permeabilization Kit (BD-bioscience). Cells were then washed and stained for intracellular markers 30 min at 4°C and acquired by FACS (BD LSR Fortessa^{™}). Data were analyzed using the FlowJo software (Treestar Inc).

**Th17 polarization *in vitro.*** OTII splenocytes were cultured in complete IMDM for 7 days under stimulation with anti-CD3/CD28 Dynabeads (4×10⁵ beads/2×10⁵ cells; Invitrogen, Thermo Fisher, Milan, IT), and in the presence of either the combination of IL-6 (20 ng/ml, PeproTech, tebu-bio, Milan, Italy), TGF-β1 (2 ng/ml, R&D Systems, Minneapolis, MN), anti-IL-4 (10 µg/ml, cat 554432, BD Biosciences) and anti-IFN-γ antibodies (10 µg/ml, cat 517904, Biolegend). Alternatively, stimulated cells were cultured in the presence of BM serum (1:25 final dilution). After 7 days OTII cells were tested for intracellular cytokine production by flow cytometry.

**Intracellular phospho-protein analysis by flow cytometry.** Vk*MYC plasma cells were stimulated with recombinant mouse IL-6 (100 ng/ml, 30 minutes), or IL-17 (50 ng/ml, 20 hours; Proleukin), or with BM serum (1:20) with or without the addition of anti-IL-17A antibodies (clone: TC11-18H10.1, 3 µg/well, Biolegend) respectively, and subsequently fixed by Cyto-Fix buffer (BD Biosciences), and permeabilized in Perm Buffer III (BD Biosciences) on ice. Staining was performed by anti-STAT3 (pY705, cat 557815, BD Biosciences) Alexa Fuor 647-conjugated antibodies and analyzed by flow cytometry.

**In vitro induction of mouse bone marrow-derived eosinophils.** Eosinophils were obtained from BM precursors as described in⁴¹. In brief, BM cells were collected from the femurs and tibiae of WT C57BL/6J mice by flushing the opened bones with PBS (Euroclone, Pero, Italy). The BM cells were cultured at 10⁶/ml in medium containing RPMI 1640 (Invitrogen) with 20% FBS (Cambrex), 100 IU/ml penicillin and 10 µg/ml streptomycin (Cellgro), 2 mM glutamine (Invitrogen), 25 mM HEPES and 1x nonessential amino acids and 1 mM sodium pyruvate (Life Technologies), and 50 µM 2-ME (Sigma-Aldrich) supplemented with 100 ng/ml stem cell factor (SCF; PeproTech) and 100 ng/ml FLT3 ligand (FLT3-L; PeproTech) from days 0 to 4. On day 4, the medium containing SCF and FLT3-L was replaced with medium containing 10 ng/ml recombinant mouse IL-5 (R&D Systems) only. On day 8, the cells were moved to new flasks and maintained in fresh medium supplemented with rmIL-5. BM eosinophils were stimulated with BM serum (1:20) with or without the addition of anti-CCR3 (CD193, Clone: J073E5, 3µg/well, cat 144503, Biolegend) or anti-IL-17A antibodies (clone: TC11-18H10.1, 3µg/well, cat 506902, Biolegend).

**BM serum cytokine quantification in mice.** Cytokines were quantified by the Myriad RBD^{™} multiplex immunoassay (Myriad RBD, Austin, TX, USA). The sera were 1:10 diluted with PBS, and stored at -80°C until sending to Myriad RBD for cytokine quantification.

**Statistics analyses and reproducibility.** Sample size was chosen taking into account the means of the target values between the experimental group and the control group, the standard error and the statistical analysis used. Based on inventors' previous experience^{14,17,33} and preliminary data obtained in the Vk*MYC and t-Vk*MYC MM models, Inventors estimated a number of 5 and 10 animals per experimental group for the *in vitro* and *in vivo* experiments, respectively, to ensure adequate power (alfa=0.05 and power=0.80) to detect significant variations in the measured events. No samples or animals were excluded from the analyses. Grubb's test was applied to exclude outliers. Animals were always matched for sex and age. Randomization was performed for *in vivo* experiments assessing the therapeutic efficacy of antibodies. No blinding was done for in vivo experiments. Data were analyzed with GraphPad Prism version 7. The data are presented as mean ± standard deviation of the mean, individual values as scatter plot with column bar graphs and were analyzed using Student's t-tests (paired or unpaired according to the experimental setting) by a two-sided and, when indicated, followed by Wilcoxon post-test. One-way ANOVA was used to compare three or more groups in time point analyses. Differences were considered significant when P < 0.05 and are indicated as NS, not significant, *P < 0.05, **P < 0.01, ***P < 0.001. Non-parametric tests were applied when variables were not normally distributed using the SPSS statistical software. N values represent biological replicates. Survival curves were compared using the Log-rank test (Mantel-Cox). All the statistics and reproducibility are reported in the figure legend. Relevant data are available from the authors.

Gene expression profiling data of primary SMM cells were obtained from⁶⁵. The probe set used for IL-17RA expression was 207707_at. Datasets were analyzed by Student's t-test directly at www.oncomine.org as 207707_at.

**Mice.** NOD and WT C57BL/6 mice were purchased from Charles River Breeding Laboratories, Calco IT. All these mice were maintained under specific pathogen-free conditions (i.e., the rodents were housed in isolated rooms, fed sterilized food and water, and routinely tested and determined free of designated pathogens capable of interfering with research objectives; SPF) in the San Raffaele facility and experiments were performed according to state guidelines and approved by the European Community Guidelines.

**Bacteria cultivation and mice infection.** *P. heparinolytica* (cat: DSM 23917, DSMZ, Germany) and *P. melaninogenica* (cat: DSM 7089, DSMZ, Germany) were cultured in Brain Heart Infusion (BHI) medium at 37 °C under anaerobic conditions, following manufacturer's instructions. Fifty microliter of bacterial growth where then transferred on chocolate agar plates and cultivated at 37 °C in AnaeroJar 2.5 L Jar System (cat: AG0025A, Thermo Fisher Scientific) and using AnaeroGen 2.5 L (OXOID; cat: 10269582, Thermo Scientific) in order to generate an anaerobic atmosphere. To infect NOD mice with *P. melaninogenica,* Ampicillin (1 g/L; cat: A0165, Sigma-Aldrich), Vancomycin (0.5 g/L; V1130, Sigma-Aldrich), and Neomycin (1 g/L; 21810-031, GIBCO) were added to the drinking water of 6 week old NOD mice and Metronidazole (2 mg/mouse; M3761, Sigma-Aldrich) was administered by oral gavage 3 times per week. Two weeks later, antibiotic-treated animals were infected by gavage with 200uL of PBS containing 10⁹ CFU of *P. melaninogenica* for 3 consecutive days/week, until the end of the experiment. Prevotella in the stool of infected mice was confirmed by PCR. For disease monitoring, mouse blood was collected and glycaemia measured once a week with CONTOUR^{®}TS device (cat: 84511129, Ascensia Diabetes Care, Italy) and CONTOUR^{®}TS strips (cat: 84511137, Ascensia Diabetes Care, Italy). Mice were considered diabetic when the glycaemia exceed 250 mg/dl for two consecutive weeks.

**Generation of dendritic cells from murine bone marrow precursors.** Bone marrow cells from 8-10-week-old C57BL/6 mouse (Charles River) were cultured in IMDM (cat BE12-722F, Lonza) 10% FBS (cat FBS-11A, Capricorn Scientific) in the presence of 25 ng/ml of recombinant murine GM-CSF (cat: 415-ML, R&D) and 5 ng/ml of recombinant murine IL-4 (cat: 404-ML, R&D) to generate dendritic cells (DCs). At days 3 and 5 DCs were split 1:2 in fresh medium containing GM-CSF and IL-4 at the described concentrations. At day 6, DC maturation was induced by overnight culture with 1ug/ml LPS from *E. coli* (cat: L6529, Sigma-Aldrich), or 1mg/ml heat killed *Prevotella heparinolytica* (cat: DSM 23917, DSMZ; P.h) or *Prevotella melaninogenica* (cat: DSM 7089, DSMZ; P.m). Vehicle: fresh medium. The next day, were assessed for intracellular IL-12 and IL-1β at FACS Canto (DB Biosciences).

**Generation of dendritic cells from human PBMCs.** CD14⁺ monocytes were isolated by magnetic sorting with anti-human CD14 microbeads (cat:130-050-201, Miltenyi) from PBMCs from a healthy donor and cultured in RPMI (cat BE15-040-CVR, Lonza) 10% FBS (cat FBS-11A, Capricorn Scientific) in the presence of 170 ng/ml of recombinant human GM-CSF (cat: 130-093-862, Miltenyi) and 24 ng/ml of recombinant human IL-4 (cat: 130-095-373, Miltenyi) to generate DCs. At day 6, DC maturation was induced either by overnight (ON) or 6 hours (6h) culture with 100ng/ml LPS from E. coli (cat: L6529, Sigma-Aldrich), or 1mg/ml of heat killed *P.h* (cat: DSM 23917, DSMZ) or *P.m.* (cat: DSM 7089, DSMZ). Vehicle: fresh medium. DCs were assessed for intracellular IL-12 and IL-1β production at FACS Canto (DB Biosciences).

**Th17 polarization *in vitro.*** Splenocytes from 8-10-week-old C57BL/6 mice were cultured in complete IMDM for 7 days under stimulation with anti-CD3/CD28 Dynabeads (4 × 10⁵ beads/2 × 10⁵ cells; cat 11452D, Invitrogen, Thermo Fisher), and in the presence of either the combination of IL-6 (20 ng/mL, PeproTech), TGF-β1 (2 ng/ mL, R&D Systems), anti-IL-4 (10 µg/mL, cat 554432, Biolegend) and anti-IFN-γ antibodies (10 µg/mL, cat 517904, Biolegend). Alternatively, stimulated cells were cultured in the presence of 1mg/ml of heat killed *P.h* or *Pm.* After 7 days cells were tested for intracellular cytokine production by flow cytometry.

**Flow cytometry.** Single cell suspensions were labeled with fluorochrome-conjugated monoclonal antibodies (either from BD Bioscience, Biolegend, or Miltenyi) after neutralization of unspecific binding with FcR blocker (BD Biosciences) and acquired by BD FACS Canto (BD Biosciences). The antibodies used for murine cells were: oIL12 (clone C15.6, cat 554479), αIL1b pro form (clone REA577, cat 130-109-044), αIL17A (clone TC11-18H10, cat 559502), αCD11b (clone M1/70, cat 101224), αCD11c (clone N418, cat 117318), αI-Ab (clone 25-9-17, cat 114406), αCD86 (clone GL1, cat 553692), αCD3 (clone 145-2C11, cat 100330), αCD8 (clone 53- 6.7, cat 560776), αCD4 (clone GK1.5, cat 553729). The antibodies used for human cells were: αCD11c (clone 3.9, cat 301641), αIL12 (clone C11.5, cat 501806), αIL1b (clone JK1B-1, cat 508206). Dead cells were excluded by ZOMBIE^{™} (1:100, Biolegend) staining. For surface staining all murine antibodies were diluted 1:200, all human antibodies were diluted 1:20, for intracellular staining murine antibodies were diluted 1:100, with the exception of αIL1b diluted 1:10, human antibodies were diluted 1:20. Data were analyzed using the FlowJo software (TreeStar Inc). Cells were also assessed for intracellular cytokine production after 6 h for IL17 at 37 °C of stimulation with 0,12 µg/ml Phorbol Myristate Acetate (PMA, cat P8139, Sigma-Aldrich)/ 2µg/ml ionomycin (cat I0634, Sigma-Aldrich). Brefeldin A (BFA, 10µg/ml, cat B7651, Sigma-Aldrich) was added to the samples during the last 5 hours for IL17 of culture. DCs were not stimulated with PMA/ionomycin but received 10µg/ml BFA after 1h of stimulation with inactivated bacteria. After incubation, cells were washed and stained for surface markers 15 min at 4 °C, fixed and permeabilized with permeabilization solution (0,5 % Saponin, cat S7900, Sigma-Aldrich, 2% FBS, cat FBS-11A, Capricorn Scientific, 0,4% sodium azide, S2002). Cells were then washed and stained for intracellular markers 30 min at 4 °C and acquired by FACS (BD LSR FortessaTM). Data were analyzed using the FlowJo software (Treestar Inc).

**Statistics analyses and reproducibility.** Data were analyzed with GraphPad Prism version 7. The data are presented as mean ± standard deviation or standard error of the mean, individual values as scatter plot with column bar graphs and were analyzed using Student's t-tests (paired or unpaired according to the experimental setting) by a two-sided and, when indicated, followed by Wilcoxon post-test. One-way ANOVA was used to compare three or more groups in time point analyses. Differences were considered significant when P < 0.05. *P < 0.05, **P < 0.01, ***P < 0.001, **** P < 0.0001. Survival curves were compared using the log-rank test (Mantel-Cox). All the statistics and reproducibility are reported in the figure 18 legend.

### RESULTS

### P. heparinolytica favors MM progression

To investigate the link between intestinal microbes and extraintestinal cancers, Vk*MYC mice, which develop a *de novo* disease mimicking MM¹⁴, were housed in animal facilities located in USA (US) and Italy (IT), and monitored within the years 2012-2018 for disease appearance and the presence of M-spike by serum protein electrophoresis¹⁴. While a monoclonal M-spike was readily detectable by 20 weeks of age in the blood of about 30% Vk*MYC mice housed in US1 and monitored before 2014, age- and sex-matched Vk*MYC mice from US2 (monitored after 2015) or IT (monitored between 2012 and 2018) did not show signs of disease for another 10-15 weeks, a time at which more than 60% of the Vk*MYC mice from the US1 colony had a detectable M-spike (Fig. 1a). Irrespective of the animal facility of origin, age- and sex-matched wild type (WT) mice, as expected¹⁵, developed M-spikes much later than Vk*MYC (Fig 1a), and never developed MM (see Fig. 2b). These findings suggested that the environment, and the microbiota in particular, has a pathogenic impact only on those mice whose plasma cells carry driver genetic alterations like the MYC activation, and is not sufficient *per se* to generate the disease in otherwise healthy mice with spontaneous monoclonal gammopathy.

To identify constituents of the microbiota, stools simultaneously collected from mice housed in the different animal facilities before 2014 and after 2015 were subjected to 16S rDNA-based amplicon sequencing. Inventors did not observe statistically significant differences between US1, US2 and IT samples in terms of intra-sample observed species (α-diversity) by Shannon or CHAO1 indexes (Fig 8). Unweighted UniFrac principal component analyses (β-diversity) clearly segregated the three cohorts of mice and showed large differences in bacterial species between US1 and US2 or IT mice, irrespective of being Vk*MYC or WT (Fig. 1b). Main diversities were found within 8 taxa (Fig. 1c), belonging to the two major phyla hosted in most mammals: Gram negative *Bacteroidetes* and Gram positive *Firmicutes*¹⁶*.* More in details, *Bacteroidetes (Bacteroidaceae, Prevoltellaceae, Rikenellaceae, and S24-7)* were more represented in the feces of US1 animals (approximately 80% in US1, 56% in US2 and 65% in IT), while US2 and IT animals were more colonized by Firmicutes *(Clostridiales;* 1.62 ± 1.3% in US1; 9.46 ± 8.7% in US2, and 7.6 ± 4% in IT). Health reports confirmed the absence of relevant pathogens in the animal facilities, US1 and US2 mice were housed at the same institution, and changes in the microbiota were not apparently related to the diet, because US1 and US2 were fed the same diet (i.e., PicoLab^{®} Rodent Diet 20 5053; LabDiet), whose content of nutrients, minerals, vitamins and calories was comparable to the diet of IT mice (Teklad Global 18% Protein Rodent Diet; Harlan). The documented changes in the microbiota were likely due instead to the different breeding strategies adopted in US1 and US2 (Fig. 1b). Whereas Vk*MYC breeding in US1 was made by crossing Vk*MYC with WT littermates, the US2 colony was generated by breeding Vk*MYC with C57BL/6J mice from the Jackson lab. Thus, the microbiota imported from the purchased mice might have modified the microbiota in the US2 colony.

Inventors sought a direct causative role of the microbiota in favoring MM development by treating IT WT mice with a combination of different wide-spectrum antibiotics (ciprofloxacin and metronidazole), and by leaving a group untreated. To perform a controlled study on genetically homogeneous tumors, antibiotic treated and untreated mice were challenged with Vk*MYC-derived Vk12598 cells, a reliable MM model (i.e., t-Vk*MYC MM; refs.^{17,18}). Antibiotic treatment was prolonged for the entire duration of the experiment, and mice were followed for M-spike appearance (Fig. 9a). As expected¹⁷, three weeks after transplantation the paraprotein was measurable in sera of 80% of untreated mice, but none of the mice treated with antibiotics showed signs of disease (Fig. 1d and Fig. 9b). This did not appear to be due to a direct effect of the antibiotics on plasma cell survival, because the M-spike appeared later in several antibiotic-treated mice (Fig. 1d). Importantly, at the time that all the untreated t-Vk*MYC MM mice with M-spike succumbed of the disease, all antibiotic-treated mice were still alive, and overall survival was improved in the latter group (Fig. 9b).

To further support the link between gut microbiota and MM progression, antibiotic-treated IT mice housed in an isolator were subjected to gavage administration of *Prevotella heparinolytica,* the *Prevotellaceae* mostly represented in US1 (Fig. 1c), before challenge with Vk12598 cells. As control, IT mice were infected with *P. melaninogenica,* which has been associated in humans with improved glucose metabolism under high-fiber diet¹⁹, and in humanized mice with aggressive type II collagen induced arthritis²⁰. While in t-Vk*MYC MM mice infected with *P. heparinolytica,* disease was accelerated, as demonstrated by reduced animal survival when compared to mock-gavaged mice, infection with *P. melaninogenica* prolonged animal survival (Fig. 1e). Thus, in these experimental conditions, microbiota constituents, and *P. heparinolytica* in particular, favor the generation of a microenvironment prone to tumor cell engraftment and expansion.

### P. heparinolytica favors induction of IL-17-producing cells

A causative link has been proposed between gut microbiota, chronic inflammation mediated by IL-17-producing cells and cancer²¹⁻²³. Interestingly, *Prevotellaceae,* which were almost only present in US1 animals (Fig. 1c), were included among the strains able to promote Th17 differentiation locally and at distant sites²⁴. Thus, Inventors searched for IL-17-producing cells in the small intestine of mice housed in the different conditions. A population of IL-17⁺ cells (Fig. 1f) was clearly detectable by flow cytometry analysis in the Peyer's patches of all examined mice (Fig. 1g and Fig. 10a and c) in the absence of overt signs of gut inflammation. The number and frequency of IL-17⁺ cells was higher in US1 than in IT mice, and was not influenced by the disease (Fig. 1g and Fig. 10a and c), thus confirming that the microbiota of US1 mice and not the pathogenic background of Vk*MYC mice favored the local expansion of IL-17-producing cells. Administration of *P. heparinolytica* but not of *P. melaninogenica* induced expansion of IL-17⁺ cells in the gut of t-Vk*MYC MM mice housed in the isolator (Fig. 1h).

To find a correlation between gut microbiota and MM, Inventors looked for IL-17-producing cells in the BM, which is the primary site of MM in both humans and Vk*MYC mice^{11,14}, of Vk*MYC mice housed in the different conditions. IL-17⁺ cells were enriched in the BM of Vk*MYC mice when compared to WT mice housed in the respective facilities (Fig. 1i and Fig. 10b and d). Accumulation of IL-17⁺ cells was more pronounced in the BM of US1 versus IT Vk*MYC mice, whereas, no difference in the number and frequency of these cells was detected in the BM of WT mice housed in either facility (Fig. 1i and Fig. 10b and d). IL-17⁺ cells were also enriched in the BM of t-Vk*MYC MM receiving *P. heparinolytica* but not of *P. melaninogenica* (Fig. 1j). Thus, a pathologic substrate is required in the BM for IL-17⁺ cell accumulation, which is favored by selected bacteria.

A direct link between gut microbiota and enrichment of IL-17-producing cells in the BM in Vk*MYC mice was suggested by the presence of a significant proportion of IL-17⁺ cells expressing the gut-homing integrin α₄β₇²⁵ in the BM of Vk*MYC housed in US1 (Fig. 1k). To prove the migration of IL-17⁺ cells from the gut into the BM of mice affected by MM, Inventors took advantage of the photoconvertible protein Kaede, which permanently changes fluorescence emission from green to red upon photoactivation, and backcrossed Kaede-transgenic mice²⁶, into IL-17A FP635 reporter knock in mice²⁷, to generate Kaede/IL-17 mice. Age- and sex-matched Kaede/IL-17 littermates were either sham-treated or injected with Vk12598 cells and monitored for disease progression. At the appearance of M-spike, the small intestine of all Kaede/IL-17 littermates was photoconverted²⁸, and the animals were euthanized 60 hours later to investigate migration of IL-17⁺ cells. Whereas the frequency of both Kaede red⁺ (i.e. IL-17⁺ cells migrated from the gut) and Kaede green⁺ (i.e. non-photoconverted IL-17⁺ cells) cells within CD3⁺CD4⁺ cells (Fig. 11) was similar in the spleen of both control and Vk12598-challenged mice (Fig. 11), Kaede red⁺ cells substantially increased in the BM of tumor-bearing mice (Fig. 1m), thus demonstrating that the presence of MM induced migration of IL-17⁺ cells from the gut to the BM.

These correlative findings prompted us to look for a causative role of microbiota-driven IL-17 in MM pathogenesis. Thus, Vk12598 cells were either injected in age- and sex-matched IL-17^{WT} or IL-17^{KO} littermates²⁹ and treated or not with antibiotics. Disease was substantially delayed in IL-17^{KO} mice when compared to WT animals (Fig. 1n). Antibiotic treatment prolonged survival of tumor bearing IL-17^{WT} mice (Fig. 1d and Fig. 1n), but had no effects on IL-17^{KO} mice (Fig. 1n), indicating that IL-17 links the microbiota to MM. These findings also confirmed that antibiotics did not have direct effects on plasma cell survival. To further support inventors' conclusion, Vk12598-challenged mice were treated with either isotype control antibodies or anti-IL17A and anti-IL17R antibodies. Treatment with anti-IL17A and anti-IL17R antibodies delayed MM development (Fig. 1o). Altogether, these findings support a direct link between microbiota diversity, and expansion of a gut-born population of IL-17⁺ cells that also preferentially accumulate in the BM of mice with MM and contribute to the pathogenesis of the disease.

### IL-17 accelerates progression of asymptomatic MM

As inventors' data, together with previous *in vitro* and *in vivo* results with human samples^{9,30-32}, suggested a role for IL-17 in favoring MM aggressiveness, Inventors backcrossed Vk*MYC mice into IL- 17^{KO} congenic mice, and monitored them for disease occurrence. Appearance of *de novo* disease was significantly delayed in Vk*MYC IL-17^{KO} mice when compared with Vk*MYC IL-17^{WT} littermates (Fig. 2a). Additionally, disease progression [i.e., M-spike ≥ 6%, which is characteristic of symptomatic, Late-MM; ref.³³] was delayed in Vk*MYC IL-17^{KO} mice (Fig. 2b) when compared to Vk*MYC IL-17^{WT} mice, thus demonstrating that IL-17 is also a precocious propeller of MM in this model. As expected, WT mice never progressed to MM (Fig. 2b).

As inventors' results suggested that IL-17 is involved in early phases of disease (Fig. 2a), Inventors quantified IL-17⁺ cells (Fig. 2c) in the BM of both asymptomatic (Early)- and symptomatic Late-MM Vk*MYC mice³³. Surprisingly, a more significant accumulation of IL-17⁺ cells was evident in the early phases of MM than in Late-MM (Fig. 2c).

Several immune cells produce IL-17⁵. Indeed, the BM of Vk*MYC mice contained measurable populations of CD3⁺CD4⁺ (Fig. 12a), CD11b⁺Gr1⁺ (Fig. 12b), Nk1.1⁺ CD90.2⁺ (Fig. 12c) and Lin⁻ CD90⁺CD127⁺ cells producing IL-17 (Fig. 12d), of which T helper type 17 (Th17) cells were the most represented (Fig. 12e). Again, a more significant accumulation of Th17 cells (Fig. 2d), and a higher ratio between Th17 cells and neoplastic plasma cells were present in the early phases of MM (Fig. 2e), thus supporting the concept that IL-17-producing cells exert a relevant pathogenic role during the asymptomatic phase and promote MM progression. BM accumulation of Th17 cells was not a characteristic of all aged mice, or a peculiarity of mice with M-spike, because WT mice, either with or without M-spike, did not show enrichment of Th17 cells (Fig. 2d).

Having found accumulation of Th17 cells in the BM of Vk*MYC mice in the early phases of MM, Inventors sought to investigate if such milieu favored Th17 differentiation. Thus, naive CD4⁺ T cells from TCR transgenic OTII mice³⁴ were cultured in the presence of BM serum from either sex- and age-matched WT or Vk*MYC mice affected by Early- or Late-MM. As control, naive CD4⁺ T cells were cultured in the presence of IL-6, TGF-β1, anti-IL-4 and anti-IFN-γ at concentrations known to induce Th17 polarization³⁵. Th17 cells were mostly induced by the BM sera from Vk*MYC mice (Fig. 3a), thus confirming that the BM becomes an ideal microenvironment for Th17 cells during disease development in Vk*MYC mice. All together, inventors' findings suggested that IL-17 has a peculiar role in the early phases of disease in Vk*MYC mice.

To mechanistically explain the role of IL-17-producing cells in MM, Inventors assessed the presence of IL-17R in Vk*MYC plasma cells by flow cytometry. As reported in human MM plasma cells³¹, Vk*MYC plasma cells (Fig 13) also expressed both subunits of the IL-17R (Fig. 3b), which was functional because exposure to saturating amounts of recombinant IL-17 induced STAT3 phosphorylation in Vk*MYC plasma cells, similarly to saturating amounts of IL-6 (Fig. 3c and e). Interestingly, IL-17 contained in the BM sera from Vk*MYC mice induced STAT3 phosphorylation, and the addition of anti-IL17 antibodies inhibited this phenomenon (Fig. 3d and e). Thus, the BM milieu of Early-MM Vk*MYC mice is rich in soluble factors favoring a Th17 switch and sustaining neoplastic plasma cells.

Because of transgene expression, all Early-MM Vk*MYC mice are bound to develop symptomatic MM¹⁴. In contrast, only a fraction of patients with SMM progresses to MM³⁶, although their plasma cells also express IL-17R (Fig. 4a). Hypothesizing that disease progression in Vk*MYC mice faithfully recapitulates MM progression in SMM patients, Inventors retrospectively measured at SMM diagnosis IL-17 levels in the BM of a cohort of patients that rapidly progressed to MM (i.e., < 3 years), and compared these data with those obtained from a cohort of SMM patients that did not progress to MM in the same time frame (Table 1). Already at the diagnosis, SMM patients progressing to MM within three years had much higher BM IL-17 than patients not progressing to MM within the same time frame (Fig. 4b). IL-17 did not further increase in MM patients either at diagnosis or after treatment (Fig. 4b). Thus, the content of IL-17 in the BM sera of SMM patients appears to be predictive of progression to symptomatic disease.

### IL-17 activates eosinophils in the BM of VK*MYC mice

BM sera of SMM patients were investigated for the content of additional inflammatory chemokines and cytokines. While IL-17 was the only one significantly increased, several other inflammatory factors attracting and activating eosinophils (i.e., RANTES, IFN-y, IL-4, IL-13, GM-CSF and IL-5) showed a trend toward enrichment in the BM sera of SMM patients rapidly progressing to MM (Fig. 14). Eosinophils play crucial roles both in plasma cell homing to the BM and their retention in the BM niche³⁷. Herein, they specifically co-localize with plasma cells³⁸, and release the proliferation inducing ligand APRIL and IL-6, essential survival factors for long-lived plasma cells³⁷. Eosinophils were indeed present in the BM of Vk*MYC IL-17^{WT} mice developing *de novo* MM, and their frequency increased with disease progression (Fig. 5a and Fig 15a). Interestingly, eosinophils were not increased in the BM of MM Vk*MYC IL-17^{KO} mice (Fig. 5a). When these cells were assessed for cytokine production, which is a marker of activation, increased frequency of IL-6⁺ eosinophils (Fig. 5b and Fig 15b) were found in the BM of Early- but not Late-MM Vk*MYC IL-17^{WT} mice (Fig. 5c). Again, the lack of IL-17 prevented eosinophil accumulation in the BM of Vk*MYC IL-17^{KO} mice affected by MM (Fig. 5c). Finally, the eosinophil mean fluorescence intensity (MFI) for IL-6 was also increased in Early-MM Vk*MYC mice (Fig. 5d), thus suggesting that eosinophil activating factors were enriched in the BM of these mice, particularly at early phases of disease. Consistently, Inventors detected a trend toward higher levels of MCP-3, which attracts and activates eosinophils³⁹, in the BM of Early-MM mice when compared to Late-MM [Early-MM 376.9 ±128.5 pg/ml (mean±SE; n=7); Late-MM 100.2 ± 15.45 pg/ml (n=5); WT 169.8 ± 46.7 (n=5); Fig. 16].

As it has been reported that cytokines of the IL-17 family induce human eosinophils to release cytokines⁴⁰, inventors investigated if IL-17 induced murine eosinophils to produce cytokines. Thus, BM precursors from WT mice were induced *in vitro* to differentiate to eosinophils [Fig. 15c and d; ref.⁴¹], and checked for IL-17 receptor expression. *In vitro* differentiated eosinophils expressed both IL-17RA and IL-17RC subunits (Fig. 5e), and upon stimulation with MCP-3 or IL-17, produced both IL-6 and TNF-α (Fig. 5f and g, respectively). Finally, inventors investigated if MCP-3 and/or IL-17 levels in the BM of Early-MM mice were sufficient to activate eosinophils. Indeed, eosinophils produced more IL-6 when cultured in the presence of BM serum from Early- than Late-MM or WT mice (Fig. 5h). This phenomenon was due to the presence of MCP-3 and IL-17 because the addition of either blocking anti-CCR3 or anti-IL-17 antibodies substantially reduced IL-6 production (Fig. 5i).

### IL-17-eosinophil axis neutralization delays MM progression

To determine whether breaking the immune axis between IL-17 and eosinophils delayed disease progression, Early-MM Vk*MYC mice were treated with a cocktail of monoclonal antibodies directed against IL-17RA, IL-17A and IL-5 (Fig. 6a), the latter being relevant for activation, recruitment and survival of eosinophils⁴². Indeed, treatment with anti-IL-5 antibodies has been shown to reduce eosinophil numbers in blood and BM of mice⁴³. Primary end point of the study was to demonstrate that the M-spike in treated mice did not reach values > 6%, as Vk*MYC mice with M-spike ≥ 6% are in the symptomatic MM phase³³. The combination of the 3 monoclonal antibodies significantly delayed disease progression, which associated with reduced accumulation of both Th17 cells (Fig. 6c) and eosinophils (Fig. 6d) in the BM of Vk*MYC mice. Interestingly, the combination of anti-IL17RA and anti-IL-17A, did not significantly impact the disease (Fig. 6b), and treatment with only anti-IL5 antibodies, while associated with reduced BM accrual of eosinophils (Fig. 17c and d), neither impacted disease progression in inventors' MM models (Fig. 6b and Fig. 17a), nor affected Th17 accrual in the BM of t-Vk*MYC MM mice (Fig. 17b). All together, these data support the concept that disease progression in Vk*MYC mice is propelled by the IL-17-eosinophil axis, which can be broken by the combination of cytokine-specific antibodies (Fig. 7).

To investigate if treatment with *Prevotella melaninogenica (Pm)* had effects also on diseases other than multiple myeloma, the inventors exploited a mouse model of diabetes (NOD mice) in which it is shown that oral administration of *Pm* delayed the appearance of diabetes when compared to mice receiving oral gavage of PBS (Fig. 18a).

To investigate the mechanism by which *Pm* and *Prevotella heparinolytica (Ph)* strains modulate Th17 expansion, inventors challenged *in vitro* bone marrow-derived dendritic cells (DCs) with the two heat-killed *Prevotella,* or *Escherichia coli*-derived LPS as control, and measured cytokine production. They found that DCs produced IL-12 in response to *E*. *coli*-derived LPS or the two *Prevotella,* thus confirming DCs were induced to maturation by interacting with commensal bacteria (Fig. 18b, c). However, *Ph* induced DCs to produce much more IL-12 and IL-1β than *Pm* (Figs. 18b and c), suggesting that *Ph*-stimulated DCs were more prone to polarize T cells toward a Th17 phenotype than *Pm*-stimulated DCs. Indeed, activation of splenocytes in the presence of *Ph* generated more Th17 cells than cultures with *Pm.* (Figs. 18d and e). These findings support the modulation of the gut microbiota to restrain Th17 skew in all those pathologies in which Th17 and IL17 are pathogenic.

### DISCUSSION

Mammals have co-evolved with their surrounding microbial environment into a complex super-organism, of which commensalism and mutualism are the most advantageous relationships. Conversely, altered host-microbiota interactions drive mucosal inflammation, autoimmunity and aerodigestive tract malignancies¹. Inventors' findings substantially extend this evidence, demonstrating that *P. heparinolytica,* a commensal bacterium, has a marked effect on the aggressiveness of extramucosal tumors, and independently of gut inflammation. Indeed, inventors provide evidence that accumulation within the BM of IL-17 producing cells, a phenomenon propelled by a commensal microbe in the absence of overt signs of gut inflammation, is a tumor cell-extrinsic mechanism driving progression of MM, and possibly other extramucosal malignancies.

Inventor's data also support the existence of a direct immunological link between the gut and the BM, and, more importantly, between the gut and the progression from asymptomatic to symptomatic MM. Thus, inventors provide mechanistic insights into what has been proposed by Enzeler and colleagues⁴⁴, who showed that antimicrobial therapy prevented solid tumor development in partially immunodeficient mice. While a substantial amount of data support a direct link between gut microbiota and gastrointestinal cancer¹, less is known on the potential role of intestinal microbes in extramucosal tumors⁴⁵. As an example, a correlation has been clearly found between orogastric infection with the pathogen *H. hepaticus* and mammary carcinoma⁴⁶, through a mechanism that requires innate immunity⁴⁷. Others have elegantly linked TLR5-signaling, microbiota, innate immunity, and extramucosal tumors²³. Thus, inventors' data extend these previous findings showing that in fully immunocompetent mice, non-pathogenic commensal microbes expand a population of IL-17 producing cells, able to migrate to the BM, and to support MM progression.

*Prevotellaceae,* which are known to promote Th17 differentiation locally and at distant sites²⁴, were almost only present in US1 animals, and *P. heparinolytica* accelerated MM progression. Thus, *Prevotella* species are primary suspects also in humans, in which the increased abundance of these bacteria at mucosal sites has been associated with Th17-mediated diseases including periodontitis²⁴ and rheumatoid arthritis⁴⁸. Interestingly, in the humanized HLA-DQ8 murine model, treatment with *P. histicola* but not *P. melaninogenica* suppressed collagen-induced rheumatoid arthritis²⁰, and P. *histicola* suppressed experimental autoimmune encephalomyelitis by modulating IL-17 production⁴⁹. On this line, an increased abundance of *Prevotella* species has been associated with reduced intestinal Th17 cell frequency and high disease activity in multiple sclerosis⁵⁰. All together, these findings suggest that selected members of the same genus have different disease modulating properties in different diseases⁵¹.

At the metagenomic level, the microbiota is rather redundant⁵², and different classes of bacteria but through similar pathways may drive cancer-promoting effects. Thus, inventors favor the hypothesis that alterations in microbial richness and function rather than true dysbiosis may affect extramucosal carcinogenesis², likely through the fine tuning of the immune response. Accordingly, at pathologic examination, inventors did not find relevant signs of inflammation in the gut of US1 animals. Rather, expansion of IL-17⁺ cells in US1 mice might be more likely driven by a different proportion of autobiont species, which are permanent members of the normal commensal microbiota¹⁶. Inventors speculate that the gut microbiota might also impact human MM. Therefore, identification of the microbiome of selected groups of cancer patients, and altering the composition of the gut microbiota, could be beneficial not only in the prevention of gastrointestinal cancer, but also in delaying progression to symptomatic MM.

Mechanistically, inventors have identified the BM milieu of Vk*MYC mice as a microenvironment rich in factors favoring eosinophils and T cells to produce cytokines promoting neoplastic plasma cell survival and expansion. It has been previously reported that IL-17 is systemically rather than locally upregulated in TLR5-unresponsive tumor-bearing mice, but only accelerates malignant progression in IL-6-unresponsive tumors²³. Inventors' findings challenge this notion, and support a promoting role for gut-driven IL-17 also in IL-6-dependent MM. As MM is an IL-6-driven neoplasm, it would be expected that in patients with TLR5^{R392X} polymorphism²³, increased IL-17 serum levels would not favor MM progression. Thus, it would be interesting to verify if in slowly progressing SMM patients TLRS^{R392X} polymorphism correlates with high serum levels of IL-17. Commensal microbes are not unique in favoring the expansion of pathogenic Th17 cells in MM. As an example, mineral oil, which is used in food, cosmetics and biomedicine, has been reported to promote plasma cells neoplasms in BALB/c mice⁵³, through IL-6⁵⁴ , eosinophils⁴³, and possibly the expansion of Th17 cells⁵⁵. Thus, inventors speculate that several environmental factors in addition to the gut microbiota substantially influence MM progression by inducing pathogenic Th17 cells. Prior studies have shown that Th17 cells are increased and serum levels of IL-17 are elevated in the BM of symptomatic MM patients^{9,30,31}, and contribute to myeloma pathology by sustaining malignant plasma cell proliferation and osteoclastogenesis^{9,31,32}. Several novel pieces of experimental evidence contained herein extend the role of Th17 cells to the asymptomatic phase of MM. Firstly, M-spike appearance was substantially delayed in Vk*MYC IL-17^{KO} mice, thus suggesting that IL-17 is needed for the correct generation of the plasma cell niche within the BM. Additionally, the frequency of Th17 cells and the ratio between Th17 cells and plasma cells in the BM were the highest in Early-MM Vk*MYC mice, which nicely correlated with increased levels of IL-17 in the BM of SMM patients that more rapidly progressed to MM. Together with the recent evidence that Th17 cells are also enriched in the BM of some MGUS-SMM patients⁵⁶, inventors' data support a much earlier role for IL-17 in this neoplasm.

The pro-tumor activity of IL-17 is not limited to its direct effect on neoplastic plasma cells expressing the IL-17R, but also through the local activation of eosinophils. Indeed, eosinophils were induced to produce TNF-α, IL-6 and likely others tumor-promoting cytokines upon stimulation with IL-17. IL-6 has long been known as a proliferative factor for MM cells. While neoplastic plasma cells can produce IL-6, the most accepted view is that the major source of this cytokine in the BM environment are BM stromal cells, osteoclasts and myeloid precursors cells from the early myeloblast to the intermediate myelocyte maturation stages⁵⁷. The latter population may contain eosinophils, which have been recently reported to support the early growth of murine neoplastic plasma cells in their BM niche⁴³. Inventors' data lend further credit to the role of eosinophils as key cells in the early neoplastic plasma cell niche, but do not exclude the role of additional IL-6-producing cells in the BM environment. The role of eosinophils in early MM is further supported by the finding that progression to MM was delayed in early-MM Vk*MYC mice only if treated with the combination of antibodies specific for IL-17, IL-17RA and IL-5, and therapeutic efficacy correlated with a reduced BM accrual of both Th17 cells and eosinophils. In more advanced MM, as the one reproduced in t-Vk*MYC MM mice, anti-IL5 antibodies are ineffective, and blocking the IL-17 signaling is sufficient to delay MM. Thus, inventors' findings confirm that eosinophils are required for the maintenance of neoplastic plasma cells in the BM niche⁴³ at the early stage of disease, and add the notion that IL-17 is one critical cytokine in the BM microenvironment that activates eosinophils to release factors supporting neoplastic plasma cells. As the role of IL-5 as growth factor for myeloma plasma cells is debated^{43,58}, and IL-5 should not impact on BM stromal cells, one mechanism by which anti-IL17, anti-IL-17RA, and anti-IL5 antibodies acted in Vk*MYC mice is a reduced accrual and survival of eosinophils and consequently of Th17 cells. While inventors' data have highlighted a relevant crosstalk between eosinophils and Th17 cells in the BM of Vk*MYC mice, other cells within the tumor microenvironment produce IL-17, and also stromal cells respond to IL-17 by producing IL-6³². Inventors' therapeutic approach should also target these cells.

Currently, the standard of care for patients with SMM has been observation until symptomatic disease occurs because of the limits in predicting disease progression¹³. At least two outputs of this study address this relevant unmet clinical need. Firstly, inventors' data suggest that in patients with SMM a high level of IL-17 in the BM predicts a faster progression to MM. Thus, IL-17 might represent an early biomarker of high-risk SMM patients¹³.

Additionally, the Food and Drug administration has recently approved the use of anti-IL-17A and anti-IL-5 antibodies for the treatment of immune-mediated diseases⁵⁹⁻⁶¹. The availability of these clinical-grade antibodies and inventors' data suggest investigating if targeting the IL-17-eosinophil immune axis would represent a potential treatment for SMM patients at high risk to progress to symptomatic MM.

The concept of host microbiota-immune system crosstalk in the pathogenesis of human diseases can be extended to immune-mediated diseases. Within this context, type 1 diabetes has been associated with gut microbiota dysbiosis (ref: PMID: 26051037) and expansion of IL-17 producing T cells (PMID: 26843788). The present results represent the first compelling evidence that administering P. *melaninogenica,* in particular by gavage, substantially delays the appearance of diabetes in NOD mice. These findings strongly support that modulation of the gut microbiota by administering *P*. *melaninogenica* or prebiotics favoring *P. melaninogenica* expansion in the gut of subjects at high risk of developing type 1 diabetes may prevent the disease. As similar mechanisms occur in type 2 diabetes (PMID: 26154056), the present findings apply to the treatment and/or prevention of type 2 diabetes, in particular in the early phase of type 2 diabetes.

### REFERENCES

1. Garrett, W.S. Cancer and the microbiota. Science 348, 80-86 (2015).
2. Schwabe, R.F. & Jobin, C. The microbiome and cancer. Nat. Rev. Cancer. 13, 800-812 (2013).
3. Belkaid, Y. & Hand, T.W. Role of the microbiota in immunity and inflammation. Cell 157, 121-141 (2014).
4. Ivanov, II, et al. Induction of intestinal Th17 cells by segmented filamentous bacteria. Cell 139, 485-498 (2009).
5. Gaffen, S.L., Jain, R., Garg, A.V. & Cua, D.J. The IL-23-IL-17 immune axis: from mechanisms to therapeutic testing. Nat. Rev. Immunol. 14, 585-600 (2014).
6. Muranski, P. & Restifo, N.P. Essentials of Th17 cell commitment and plasticity. Blood 121, 2402-2414 (2013).
7. McAllister, F., et al. Oncogenic Kras activates a hematopoietic-to-epithelial IL-17 signaling axis in preinvasive pancreatic neoplasia. Cancer Cell 25, 621-637 (2014).
8. Tartour, E., et al. Interleukin 17, a T-cell-derived cytokine, promotes tumorigenicity of human cervical tumors in nude mice. Cancer Res. 59, 3698-3704 (1999).
9. Noonan, K., et al. A novel role of IL-17-producing lymphocytes in mediating lytic bone disease in multiple myeloma. Blood 116, 3554-3563 (2010).
10. Wang, L., et al. IL-17 can promote tumor growth through an IL-6-Stat3 signaling pathway. J. Exp. Med. 206, 1457-1464 (2009).
11. Palumbo, A. & Anderson, K. Multiple myeloma. N. Engl. J. Med. 364, 1046-1060 (2011).
12. Kyle, R.A., et al. Clinical course and prognosis of smoldering (asymptomatic) multiple myeloma. N. Engl. J. Med. 356, 2582-2590 (2007).
13. Rajkumar, S.V., Landgren, O. & Mateos, M.V. Smoldering multiple myeloma. Blood 125, 3069-3075 (2015).
14. Chesi, M., et al. AID-dependent activation of a MYC transgene induces multiple myeloma in a conditional mouse model of post-germinal center malignancies. Cancer Cell 13, 167-180 (2008).
15. Radl, J. & Hollander, C.F. Homogeneous immunoglobulins in sera of mice during aging. J. Immunol. 112, 2271-2273 (1974).
16. Ivanov, II & Honda, K. Intestinal commensal microbes as immune modulators. Cell Host Microbe 12, 496-508 (2012).
17. Chesi, M., et al. Drug response in a genetically engineered mouse model of multiple myeloma is predictive of clinical efficacy. Blood 120, 376-385 (2012).
18. Chesi, M., et al. IAP antagonists induce anti-tumor immunity in multiple myeloma. Nat. Med. 22, 1411-1420 (2016).
19. Kovatcheva-Datchary, P., et al. Dietary Fiber-Induced Improvement in Glucose Metabolism Is Associated with Increased Abundance of Prevotella. Cell Metab. 22, 971-982 (2015).
20. Marietta, E.V., et al. Suppression of Inflammatory Arthritis by Human Gut-Derived Prevotella histicola in Humanized Mice. Arthritis & rheumatology 68, 2878-2888 (2016).
21. Wu, S., et al. A human colonic commensal promotes colon tumorigenesis via activation of T helper type 17 T cell responses. Nat. Med. 15, 1016-1022 (2009).
22. Grivennikov, S.I., et al. Adenoma-linked barrier defects and microbial products drive IL-23/IL-17-mediated tumour growth. Nature 491, 254-258 (2012).
23. Rutkowski, M.R., et al. Microbially driven TLR5-dependent signaling governs distal malignant progression through tumor-promoting inflammation. Cancer Cell 27, 27-40 (2015).
24. de Aquino, S.G., et al. Periodontal pathogens directly promote autoimmune experimental arthritis by inducing a TLR2- and IL-1-driven Th17 response. J. Immunol. 192, 4103-4111 (2014).
25. Wagner, N., et al. Critical role for beta7 integrins in formation of the gut-associated lymphoid tissue. Nature 382, 366-370 (1996).
26. Tomura, M., et al. Monitoring cellular movement in vivo with photoconvertible fluorescence protein "Kaede" transgenic mice. Proc. Natl. Acad. Sci. U. S. A. 105, 10871-10876 (2008).
27. Gagliani, N., et al. Th17 cells transdifferentiate into regulatory T cells during resolution of inflammation. Nature 523, 221-225 (2015).
28. Krebs, C.F., et al. Autoimmune Renal Disease Is Exacerbated by S1P-Receptor-1-Dependent Intestinal Th17 Cell Migration to the Kidney. Immunity 45, 1078-1092 (2016).
29. Nakae, S., et al. Antigen-specific T cell Sensitization is impaired in IL-17-deficient mice, causing suppression of allergic cellular and humoral responses. Immunity 17, 375-387 (2002).
30. Dhodapkar, K.M., et al. Dendritic cells mediate the induction of polyfunctional human IL17-producing cells (Th17-1 cells) enriched in the bone marrow of patients with myeloma. Blood 112, 2878-2885 (2008).
31. Prabhala, R.H., et al. Elevated IL-17 produced by TH17 cells promotes myeloma cell growth and inhibits immune function in multiple myeloma. Blood 115, 5385-5392 (2010).
32. Prabhala, R.H., et al. Targeting IL-17A in multiple myeloma: a potential novel therapeutic approach in myeloma. Leukemia 30, 379-389 (2016).
33. Calcinotto, A., et al. Modifications of the mouse bone marrow microenvironment favor angiogenesis and correlate with disease progression from asymptomatic to symptomatic multiple myeloma. Oncoimmunology 4, e1008850 (2015).
34. Barnden, M.J., Allison, J., Heath, W.R. & Carbone, F.R. Defective TCR expression in transgenic mice constructed using cDNA-based alpha- and beta-chain genes under the control of heterologous regulatory elements. Immunol Cell Biol 76, 34-40 (1998).
35. Aggarwal, S., Ghilardi, N., Xie, M.H., de Sauvage, F.J. & Gurney, A.L. Interleukin-23 promotes a distinct CD4 T cell activation state characterized by the production of interleukin-17. J. Biol. Chem. 278, 1910-1914 (2003).
36. Agarwal, A. & Ghobrial, I.M. Monoclonal gammopathy of undetermined significance and smoldering multiple myeloma: a review of the current understanding of epidemiology, biology, risk stratification, and management of myeloma precursor disease. Clin. Cancer Res. 19, 985-994 (2013).
37. Chu, V.T., et al. Eosinophils are required for the maintenance of plasma cells in the bone marrow. Nat. Immunol. 12, 151-159 (2011).
38. Zehentmeier, S., et al. Static and dynamic components synergize to form a stable survival niche for bone marrow plasma cells. Eur. J. Immunol. 44, 2306-2317 (2014).
39. Dahinden, C.A., et al. Monocyte chemotactic protein 3 is a most effective basophil- and eosinophil-activating chemokine. J. Exp. Med. 179, 751-756 (1994).
40. Cheung, P.F., Wong, C.K. & Lam, C.W. Molecular mechanisms of cytokine and chemokine release from eosinophils activated by IL-17A, IL-17F, and IL-23: implication for Th17 lymphocytes-mediated allergic inflammation. J. Immunol. 180, 5625-5635 (2008).
41. Dyer, K.D., et al. Functionally competent eosinophils differentiated ex vivo in high purity from normal mouse bone marrow. J. Immunol. 181, 4004-4009 (2008).
42. Rosenberg, H.F., Dyer, K.D. & Foster, P.S. Eosinophils: changing perspectives in health and disease. Nat. Rev. Immunol. 13, 9-22 (2013).
43. Wong, D., et al. Eosinophils and megakaryocytes support the early growth of murine MOPC315 myeloma cells in their bone marrow niches. PLoS One 9, e109018 (2014).
44. Enzler, T., et al. Deficiencies of GM-CSF and interferon gamma link inflammation and cancer. J. Exp. Med. 197, 1213-1219 (2003).
45. Rutkowski, M.R. & Conejo-Garcia, J.R. Size does not matter: commensal microorganisms forge tumor-promoting inflammation and anti-tumor immunity. Oncoscience 2, 239-246 (2015).
46. Rao, V.P., et al. Innate immune inflammatory response against enteric bacteria Helicobacter hepaticus induces mammary adenocarcinoma in mice. Cancer Res. 66, 7395-7400 (2006).
47. Lakritz, J.R., et al. Gut bacteria require neutrophils to promote mammary tumorigenesis. Oncotarget 6, 9387-9396 (2015).
48. Scher, J.U., et al. Expansion of intestinal Prevotella copri correlates with enhanced susceptibility to arthritis. eLife 2, e01202 (2013).
49. Mangalam, A., et al. Human Gut-Derived Commensal Bacteria Suppress CNS Inflammatory and Demyelinating Disease. Cell Rep 20, 1269-1277 (2017).
50. Cosorich, I., et al. High frequency of intestinal TH17 cells correlates with microbiota alterations and disease activity in multiple sclerosis. Science advances 3, e1700492 (2017).
51. Ley, R.E. Gut microbiota in 2015: Prevotella in the gut: choose carefully. Nat Rev Gastroenterol Hepatol 13, 69-70 (2016).
52. Human Microbiome Project, C. Structure, function and diversity of the healthy human microbiome. Nature 486, 207-214 (2012).
53. Potter, M. & Boyce, C.R. Induction of plasma-cell neoplasms in strain BALB/c mice with mineral oil and mineral oil adjuvants. Nature 193, 1086-1087 (1962).
54. Suematsu, S., et al. Generation of plasmacytomas with the chromosomal translocation t(12; 15) in interleukin 6 transgenic mice. Proc. Natl. Acad. Sci. U. S. A. 89, 232-235 (1992).
55. Yau, A.C.Y., Lonnblom, E., Zhong, J. & Holmdahl, R. Influence of hydrocarbon oil structure on adjuvanticity and autoimmunity. Sci. Rep. 7, 14998 (2017).
56. Di Lullo, G., Marcatti, M. & Protti, M.P. Non-redundant roles for Th17 and Th22 cells in multiple myeloma clinical correlates. Oncoimmunology (2015*).*
57. Matthes, T., Manfroi, B. & Huard, B. Revisiting IL-6 antagonism in multiple myeloma. Crit Rev Oncol Hematol 105, 1-4 (2016).
58. Anderson, K.C., Jones, R.M., Morimoto, C., Leavitt, P. & Barut, B.A. Response patterns of purified myeloma cells to hematopoietic growth factors. Blood 73, 1915-1924 (1989).
59. Langley, R.G., et al. Secukinumab in plaque psoriasis--results of two phase 3 trials. N. Engl. J. Med. 371, 326-338 (2014).
60. Bel, E.H., et al. Oral glucocorticoid-sparing effect of mepolizumab in eosinophilic asthma. N. Engl. J. Med. 371, 1189-1197 (2014).
61. Ortega, H.G., et al. Mepolizumab treatment in patients with severe eosinophilic asthma. N. Engl. J. Med. 371, 1198-1207 (2014).
62. Shou, Y., et al. Diverse karyotypic abnormalities of the c-myc locus associated with c-myc dysregulation and tumor progression in multiple myeloma. Proc. Natl. Acad. Sci. U. S. A. 97, 228-233 (2000).
63. Affer, M., et al. Promiscuous MYC locus rearrangements hijack enhancers but mostly super-enhancers to dysregulate MYC expression in multiple myeloma. Leukemia 28, 1725-1735 (2014).
64. Elinav, E., et al. NLRP6 inflammasome regulates colonic microbial ecology and risk for colitis. Cell 145, 745-757 (2011).
65. Zhan, F., et al. CKS1B, overexpressed in aggressive disease, regulates multiple myeloma growth and survival through SKP2- and p27Kip1-dependent and -independent mechanisms. Blood 109, 4995-5001 (2007).

## Claims

1. At least one bacterial strain of *Prevotella melaninogenica* for use in the treatment and/or prevention of cancer wherein said cancer is caused by a tumor cell expressing IL-17 or expressing the receptor for IL-17.

2. At least one bacterial strain of *Prevotella melaninogenica* for use in the prevention of diabetes, preferably Type 1 diabetes.

3. The at least one bacterial strain of *Prevotella melaninogenica* for use according to claim 1 wherein the cancer is selected from the group consisting of: multiple myeloma (MM), bladder cancer, brain and CNS cancer, breast cancer, cervical cancer, colorectal cancer, esophageal cancer, gastric cancer, gastro-intestinal cancer, head and neck cancer, kidney cancer, liver cancer, lung cancer, lymphoma, ovarian cancer, pancreatic cancer, prostate cancer, chronic lymphocytic leukemia preferably
wherein said strain delays the development of MM in a subject affected by monoclonal gammopathy of undetermined significance (MGUS) or smoldering MM (SMM).

4. The at least one bacterial strain of *Prevotella melaninogenica* for use according to claim 3 wherein the cancer is multiple myeloma (MM).

5. The at least one bacterial strain of *Prevotella melaninogenica* for use according to any one of previous claim wherein said strain is isolated from a biological sample, alive, sporulated, encapsulated, genetically modified or lyophilized.

6. A composition comprising at least one bacterial strain of *Prevotella melaninogenica* and at least one pharmaceutical acceptable carrier, for use in the treatment of cancer wherein said cancer is caused by a tumor cell expressing IL-17 or expressing the receptor for IL-17 or in the prevention of diabetes.

7. The composition for use according to claim 6 wherein the diabetes is Type I diabetes and wherein the cancer is selected from the group consisting of: multiple myeloma (MM), bladder cancer, brain and CNS cancer, breast cancer, cervical cancer, colorectal cancer, esophageal cancer, gastric cancer, gastro-intestinal cancer, head and neck cancer, kidney cancer, liver cancer, lung cancer, lymphoma, ovarian cancer, pancreatic cancer, prostate cancer, chronic lymphocytic leukemia preferably wherein said strain delays the development of MM in a subject affected by monoclonal gammopathy of undetermined significance (MGUS) or smoldering MM (SMM).

8. The composition for use according to claims 6 and 7 wherein said composition comprises between 1×10⁷ to 1×10¹² *Prevotella melaninogenica.*

9. The at least one bacterial strain of *Prevotella melaninogenica* or the composition for use according to any one of previous claim wherein said strain of *Prevotella melaninogenica* is *Prevotella melaninogenica* deposited with the accession number DSM 7089 or DSM-26980 in the DSMZ bank.

10. The composition for use according to any one of claim 6 to 9, wherein the pharmaceutical composition is a nutraceutical or a food.

## Patentansprüche

1. Mindestens ein Bakterienstamm von *Prevotella melaninogenica* zur Verwendung bei der Behandlung und/oder Vorbeugung von Krebs, wobei der Krebs durch eine Tumorzelle verursacht wird, die IL-17 exprimiert oder den Rezeptor für IL-17 exprimiert.

2. Mindestens ein Bakterienstamm von *Prevotella melaninogenica* zur Verwendung bei der Vorbeugung von Diabetes, vorzugsweise Typ-1-Diabetes.

3. Mindestens ein Bakterienstamm von *Prevotella melaninogenica* zur Verwendung nach Anspruch 1, wobei der Krebs ausgewählt ist aus der Gruppe bestehend aus:
Multiplem Myelom (MM), Blasenkrebs, Gehirn- und ZNS-Krebs, Brustkrebs, Gebärmutterhalskrebs, Darmkrebs, Speiseröhrenkrebs, Magenkrebs, Magen-Darm-Krebs, Kopf- und Halskrebs, Nierenkrebs, Leberkrebs, Lungenkrebs, Lymphom, Eierstockkrebs, Bauchspeicheldrüsenkrebs, Prostatakrebs, chronischer lymphatischer Leukämie,
wobei vorzugsweise der Stamm die Entwicklung von MM in einem Subjekt verzögert, das an monoklonaler Gammopathie unklarer Signifikanz (MGUS) oder schwelendem MM (SMM) leidet.

4. Mindestens ein Bakterienstamm von *Prevotella melaninogenica* zur Verwendung nach Anspruch 3, wobei der Krebs ein multiples Myelom (MM) ist.

5. Mindestens ein Bakterienstamm von *Prevotella melaninogenica* zur Verwendung nach einem der vorhergehenden Ansprüche, wobei der Stamm aus einer biologischen Probe isoliert, lebend, sporuliert, eingekapselt, genetisch modifiziert oder lyophilisiert ist.

6. Zusammensetzung, die mindestens einen Bakterienstamm von *Prevotella melaninogenica* und mindestens einen pharmazeutisch verträglichen Träger enthält, zur Verwendung bei der Behandlung von Krebs, wobei der Krebs durch eine Tumorzelle verursacht wird, die IL-17 exprimiert oder den Rezeptor für IL-17 exprimiert, oder bei der Vorbeugung von Diabetes.

7. Zusammensetzung zur Verwendung nach Anspruch 6, wobei es sich bei dem Diabetes um Typ-I-Diabetes handelt und wobei der Krebs ausgewählt ist aus der Gruppe, bestehend aus: Multiplem Myelom (MM), Blasenkrebs, Gehirn- und ZNS-Krebs, Brustkrebs, Gebärmutterhalskrebs, Darmkrebs, Speiseröhrenkrebs, Magenkrebs, Magen-Darm-Krebs, Kopf- und Halskrebs, Nierenkrebs, Leberkrebs, Lungenkrebs, Lymphom, Eierstockkrebs, Bauchspeicheldrüsenkrebs, Prostatakrebs, chronischer lymphatischer Leukämie, wobei der Stamm vorzugsweise die Entwicklung von MM in einem Subjekt verzögert, das an monoklonaler Gammopathie unklarer Signifikanz (MGUS) oder schwelendem MM (SMM) leidet.

8. Zusammensetzung zur Verwendung nach den Ansprüchen 6 und 7, wobei die Zusammensetzung zwischen 1 × 10⁷ bis 1 × 10¹² *Prevotella melaninogenica* enthält.

9. Mindestens ein Bakterienstamm von *Prevotella melaninogenica* oder die Zusammensetzung zur Verwendung nach einem der vorhergehenden Ansprüche, wobei es sich bei dem Stamm von *Prevotella melaninogenica* um *Prevotella melaninogenica* handelt, der mit der Hinterlegungsnummer DSM 7089 oder DSM-26980 in der DSMZ-Bank hinterlegt ist.

10. Zusammensetzung zur Verwendung nach einem der Ansprüche 6 bis 9, wobei die pharmazeutische Zusammensetzung ein Nutrazeutikum oder ein Lebensmittel ist.

## Revendications

1. Au moins une souche bactérienne de *Prevotella melaninogenica* pour une utilisation dans le traitement et/ou la prévention du cancer, dans laquelle ledit cancer est provoqué par une cellule tumorale exprimant IL-17 ou exprimant le récepteur pour IL-17.

2. Au moins une souche bactérienne de *Prevotella melaninogenica* pour une utilisation dans la prévention du diabète, de préférence le diabète de type 1.

3. Au moins une souche bactérienne de *Prevotella melaninogenica* pour une utilisation selon la revendication 1, dans laquelle le cancer est choisi dans le groupe constitué par : le myélome multiple (MM), le cancer de la vessie, le cancer du cerveau et du système nerveux central, le cancer du sein, le cancer de l'utérus, le cancer colorectal, le cancer de l'œsophage, le cancer de l'estomac, le cancer gastro-intestinal, le cancer de la tête et du cou, le cancer du rein, le cancer du foie, le cancer du poumon, le lymphome, le cancer des ovaires, le cancer du pancréas, le cancer de la prostate, la leucémie lymphoïde chronique, de préférence dans laquelle ladite souche retarde le développement du MM chez un sujet atteint de gammapathie monoclonale de signification indéterminée (GMSI) ou de MM indolent (MMI).

4. Au moins une souche bactérienne de *Prevotella melaninogenica* pour une utilisation selon la revendication 3, dans laquelle le cancer est le myélome multiple (MM).

5. Au moins une souche bactérienne de *Prevotella melaninogenica* pour une utilisation selon l'une quelconque des revendications précédentes, dans laquelle ladite souche est isolée d'un échantillon biologique, vivante, sporulée, encapsulée, génétiquement modifiée ou lyophilisée.

6. Composition comprenant au moins une souche bactérienne de *Prevotella melaninogenica* et au moins un support pharmaceutique acceptable, pour une utilisation dans le traitement du cancer, dans laquelle ledit cancer est provoqué par une cellule tumorale exprimant IL-17 ou exprimant le récepteur pour IL-17, ou dans la prévention du diabète.

7. Composition pour une utilisation selon la revendication 6, dans laquelle le diabète est le diabète de type I et dans laquelle le cancer est choisi dans le groupe constitué par : le myélome multiple (MM), le cancer de la vessie, le cancer du cerveau et du système nerveux central, le cancer du sein, le cancer de l'utérus, le cancer colorectal, le cancer de l'œsophage, le cancer de l'estomac, le cancer gastro-intestinal, le cancer de la tête et du cou, le cancer du rein, le cancer du foie, le cancer du poumon, le lymphome, le cancer des ovaires, le cancer du pancréas, le cancer de la prostate, la leucémie lymphoïde chronique, de préférence dans laquelle ladite souche retarde le développement du MM chez un sujet atteint de gammapathie monoclonale de signification indéterminée (GMSI) ou de MM indolent (MMI).

8. Composition pour une utilisation selon les revendications 6 et 7, dans laquelle ladite composition comprend entre 1 x 10⁷ et 1 x 10¹² *Prevotella melaninogenica.*

9. Au moins une souche bactérienne de *Prevotella melaninogenica* ou composition pour une utilisation selon l'une quelconque des revendications précédentes, dans laquelle ladite souche de *Prevotella melaninogenica* est *Prevotella melaninogenica* déposée sous le numéro d'accès DSM 7089 ou DSM-26980 dans la banque DSMZ.

10. Composition pour une utilisation selon les revendications 6 à 9, dans laquelle la composition pharmaceutique est un nutraceutique ou un aliment.
